(19) Europäisches Patentamt
European Patent Office
Office européen des brevets

(11) Publication number: **0 082 252**
**B1**

(12) # EUROPEAN PATENT SPECIFICATION

(45) Date of publication of patent specification: **21.08.85**

(21) Application number: **82108694.9**

(22) Date of filing: **20.09.82**

(51) Int. Cl.[4]: **C 07 C 17/26**, C 07 C 29/62, C 07 C 31/38, C 07 C 33/42, C 07 C 33/46, C 07 C 33/48, C 07 C 37/62, C 07 C 39/26, C 07 C 45/68, C 07 C 49/15, C 07 C 49/175

(54) **Process for producing fluorine-containing organic compound.**

(30) Priority: **26.11.81 JP 189458/81**
**26.11.81 JP 189459/81**
**08.03.82 JP 36129/82**

(43) Date of publication of application:
**29.06.83 Bulletin 83/26**

(45) Publication of the grant of the patent:
**21.08.85 Bulletin 85/34**

(84) Designated Contracting States:
**DE FR GB**

(56) References cited:
**DE-B-2 613 003**
**US-A-3 171 861**

**JOURNAL OF ORGANOMETALLIC CHEMISTRY, vol. 140, no. 1, 1977. G. SANTINI et al. "Reactions of perfluoroalkylcalcium derivatives with ketones and aldehydes", pages 1—9**

**HOUBEN-WEYL "Methoden der organischen Chemie", vol. V 3, 1962, 4th edition, GEORG THIEME VERLAG, Stuttgart, pages 319-321**

**1981, Columbus, Ohio, USA. T. KITAZUME et al. "Perfluoroalkyltin (IV) halides: a novel perfluoroalkylating agent for carbonyl compounds", page 573, column 2, abstract no. 202714h**

(73) Proprietor: **Daikin Kogyo Co., Ltd.**
**Shinhankyu Building No 1-12-39, Umeda Kita-ku**
**Osaka-shi Osaka-fu (JP)**

(72) Inventor: **Ishikawa, Nobuo**
**c/o Tokyo Techn. University**
**12-1, Ohokayama 2-chome Meguro-ku Tokyo (JP)**
Inventor: **Kitazume, Tomoya**
**c/o Tokyo Techn. University**
**12-1, Ohokayama 2-chome Meguro-ku Tokyo (JP)**

(74) Representative: **Patentanwälte TER MEER - MÜLLER - STEINMEISTER**
**Mauerkircherstrasse 45**
**D-8000 München 80 (DE)**

(56) References cited:

**Columbus, Ohio, USA. A. SEKIYA et al. "Reaction of heptafluoro-1-methylethylzinc iodide with halides and anhydrides of carboxylic acids", page 519, column 2, abstract no. 139560b**

**December 1972, Columbus, Ohio, USA. V. SOLOVEVA et al. "Activity of hydrogenation catalysts in an ultrasonic field", page 357, column 1, abstract no. 163979e**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 082 252 B1

**Description**

Background of the Invention

Title of the Invention

Process for producing fluorine-containing organic compound.

Field of the Invention

This Invention relates to a process for producing fluorine-containing organic compounds.

Description of the Prior Art

Compounds containing fluorine have versatile useful applications by virtue of their superior stability, chemical resistance, weather resistance, water and oil repellent properties, physiological activity and so on. A variety of studies have been made on the compounds containing the fluorine-containing alkyl group within their molecules, and several new synthesizing methods have been hitherto developed. There are reported of the synthesizing methods; for the compounds containing trifluoromethyl group, the followings may be mentioned, (1) a method to directly fluorinate the methyl group with metallic fluorides, (2) an halogen exchange reaction on $CCl_3$ group with HF—$SbCl_5$, (3) a method to fluorinate COOH group with $SF_4$, (4) radical reaction to introduce a $CF_3$ group into heterocyclic compounds, (5) a method to substitute iodine which is introduced into aromatic compounds for Rf by means of RfCuI (Ullmann-type reaction) (RF indicates fluorine-containing aliphatic group; hereinafter so represents). Further, it is known that the trifluoromethyl mangesium iodide ($CF_3MgI$) can not be used for trifluoromethylation reaction in that the compound is very unstable due to its property to eliminate $MgF_2$ by readily extracting F because its constituent Mg is highly bondable with F.

Among above-described synthesizing methods for trifluoromethyl-containing compounds, the methods other than the Ullmann-type reaction, the reagent for fluorination is highly hazardous, and that its handling is difficult, and that it is hard to optionally introduce the trifluoromethyl group into a desired position in a molecule.

Conversely, there is reported that β-keto carboxylic acid ester can be synthesized by applying the Reformatsky reaction on ester compounds.

However, the reaction is difficult and least successful instances are known upto present. The details are explained as follows:

Firstly, α-carboxylic acid ester (*1*) is caused to react with zinc in accordance with the following reaction, then a Reformatsky-type reagent (*2*) is obtained.

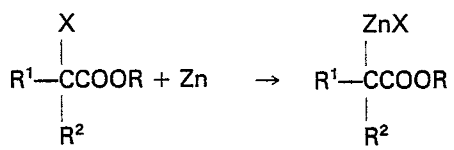

*1* ($R^1$, $R^2$ : alkyl group, X: Br)    *2* (R: alkyl group)

In this case, as it is not desirable when the raw material easter (*1*) and the product, ester (*2*) are condensated, and

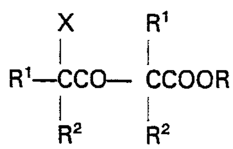

is produced, it was a prerequisite to handle them without generating the above result (or preventing the reaction of both esters).

Accordingly, in obtaining an end product β-ketocarboxylic acid ester (*4*) by subjecting the above-described reagent (2) to reaction with a ester compound (*3*) with the following formula, due to the above prerequisite, it becomes difficult to cause the reaction itself of the ester (*2*) with the ester (*3*).

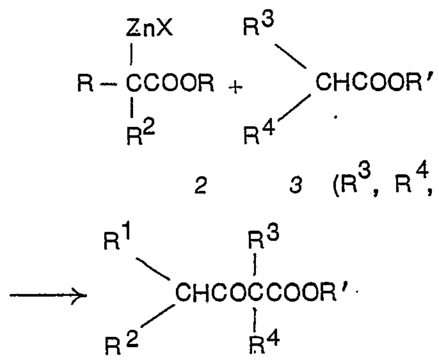

2 3 ($R^3$, $R^4$, R'·: alkyl group)

Thus, it is necessary to select an ester that may easily react with the reagent (*2*) without self-condensation between the ester (*1*) and (*2*).

This is a reason why there have been almost no successful experimental results in the synthesizing of the compound as intended.

In consequence, even when the fluorine-containing ester which is represented by $R_fCOOR'$ ($R_f$: fluorine-containing alkyl group is employed as the above-described ester compound (*3*) so as to introduce fluorine into the object compound, it is not easy to obtain a fluorine-containing alkyl keto carboxylic acid ester by means of the said Reformatsky reaction.

OBJECTS AND SUMMARY OF THE INVENTION

Accordingly, the primary object of this invention is to present a method that can proceed the reaction for introducing the fluorine-containing group into the organic compound in an easy and safety under the normal conditions.

The secondary object of the invention is to present a method to synthesize organic compounds containing a fluorine-containing aliphatic group such as trifluoromethyl group in particular with good yield and in a stabilized fashion.

Further, the third object of the invention is to present a method than can introduce various RfCO groups into the intended compounds at the specified position preferentially and with a high yield rate under mild conditions by applying the Reformatsky reaction.

The process of the present invention to produce fluorine-containing organic compounds is characterized in the introduction of Rf group into the above-said organic compounds by subjecting the fluorine-containing aliphatic iodides which are represented by $R_fI$, $R_fZnI$, or $R_fSnX_2I$ (where $R_f$ indicates the fluorine-containing aliphatic group; X indicates halogen), and organic compounds to reaction under the action of ultrasonic waves.

The present invention is related to the process for producing fluorine-containing diketones, and characterized, as an application of the Reformatsky reaction, to have (1) a process to generate a metallic halogenated ketone which is represented by the formula:

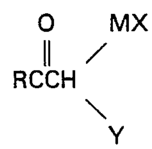

(where R: aliphatic hydrocarbon-oxy group, or aliphatic hydrocarbon group, or fluoro aliphatic hydrocarbon group which may form a ring with the carbon atoms being bonded with X; X: halogen; Y: hydrogen atom or fluorine atom; and M: zinc or magnesium) by causing the halogenated ketone represented by the Formula:

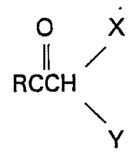

(where R, X and Y are same as the above difinition) to reaction with the metal consisting of zinc or magnesium, and to have (2) a process to generate the fluorine-containing diketones which represented by the Formula:

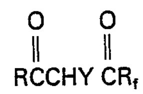

(where R and Y are as described before, and $R_f$ is fluoroaliphatic hydrocarbon group and includes those containing ether bonds within the chain), by causing the metallic halogenated ketone to react with the fluoroester which is represented by the Formula:

$R_fCOOR'$

(where $R_f$ is as described above, and R' is aliphatic hydrocarbon group or aromatic hydrocarbon group), under the action of ultrasonic waves.

DESCRIPTION OF THE PREFERRED EMBODIMENT

The method of the present invention is exemplified as follows.
The fluorine-containing aliphatic iodide which is represented by the Formula:

$R_fI$

(where $R_f$ indicates the fluorine-containing aliphatic group), and the carbonylic compound which is represented by the Formula:

RCOR'

(where R and R': similar or dissimilar atom or group selected from the group comprising the hydrogen atom, aliphatic group and aromatic group, and can form a ring jointly where both of them are of aliphatic groups), are caused to react under the zinc powder and the halogenated tin powder which is represented by the Formula:

$SnX_2$

(where X indicates the halogen like chlorine, bromine and iodine, etc.).

by being subjected to the action of ultrasonic waves. (where the halogenated tin powder is employed, it is effective under the addition of the tertiary amine, in place of the environment under the action of ultrasonic waves). Following the above reaction, the fluorine-containing carbinol which is represented by the Formula:

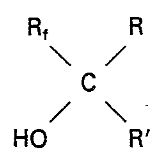

(where $R_f$, R and R' are the same as above), by subjecting the reaction product to hydrolysis.

Namely, the reaction in accordance with the present invention is capable to synthesize the fluorine-containing carbinol with a high yield and in a sabilized fashion, when the raw material RfI and RCOR' undergo reaction, preferably under the presence of the zinc powder and/or the halogenated tin powder, within a reactor, under the action of ultrsonic waves, by causing the interaction between molecules, and thereby cross-coupling RCOR' with $R_fI$, and thereafter causing hydrolysis. This reaction is conjectured that, for instance, where the zinc powder is employed (it is similar with the halogenated tin powder), the reaction may proceed as follows:

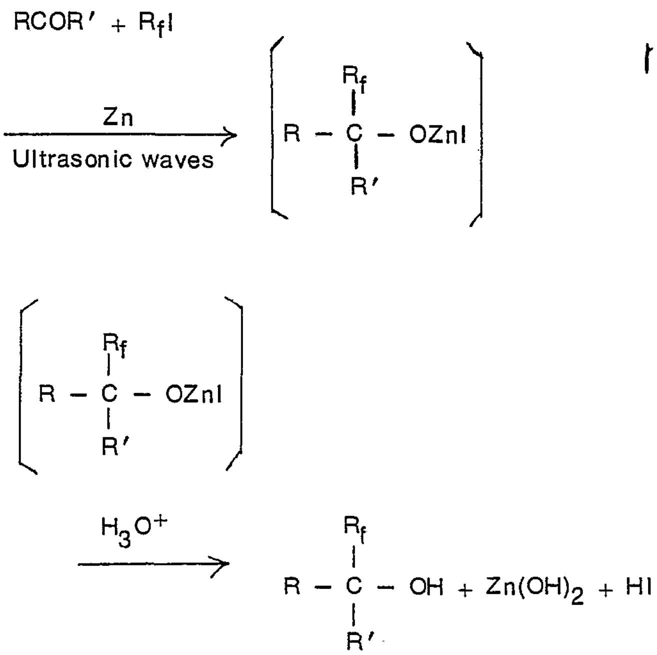


In this case, it is thought that the above-said intermediate product may be produced, in that the Zn powder (and/or halogenated tin powder) produces a stabilized $R_fZnI$ (and/or $R_fSnX_2I$) by first reacting with the reaction substance, particularly $R_fI$, and then the product is caused to have a full interaction with RCOR'.

Namely, with the method of this invention, although the mechanism is not fully clarified as present, it is thought that the interaction between the reaction molecules is made closer with the action of ultrasonic waves as given above, and thereby accelerates the generation of the above-mentioned intermediate product.

Where the halogenated tin is employed, it is also effective to cause the reaction under the addition of the tertiary amine (i.e., pyridine, triethylamine, N,N-dimethylaniline) instead of the ultrasonic wave. It is thought that the tertiary amine is coordinated at Sn position of $R_fSnIX_2$ and activates the $R_f$-Sn bonding.

Where the ultrasonic wave or the tertiary amine is not caused to act, it is experimentally confirmed that the reaction does not proceed entirely.

What is important with the method of this invention is represented by the employment of Zn and/or $SnX_2$ (where X: halogens such as chlorine, bromine and iodine, etc.) for the above-said cross coupling. This reaction appears to be similar to the publicly known Grignard reaction.

However, where Mg or Li is employed, $R_fMgI$ (or $R_fLi$) itself is extremely unstable to produce the above-said intermediate product, thereby it precludes its use for the synthesizing reagent. In other words, Mg or Li has a strong affinity toward F. Where Mg (or Li) is caused to react with $R_fI$, $MgF_2$ or LiF is eliminated from $R_fMgI$ or $R_f$ Li, resulting in the production of fluoroalkene. Thus the Grignard reagent (or $R_fLi$) such as $R_fMgI$ can not be used for the present invention.

This fact indicates the characteristics or speciality of the present invention which introduces fluorine-containing aliphatic group. Furthermore, with the method of this invention, the raw material are stabilized respectively, and also the reaction can be implemented within a single reaction vessel under the normal conditions subject to the ambient temperature and atmospheric pressure. Thereby easier handling is available and most practicable.

For the method of this invention, as the usable $R_fI$, the fluorine-containing aliphatic iodines which are represented by the Formula, $CF_3(CF_2)_nI$ or $(CF_3)_2CF(CF_2)_nI$ can be counted. These include: $CF_2I$, $CF_3CF_2I$, $CF_3(CF_2)_2I$, $CF_3(CF_2)_3I$, $CF_3(CF_2)_4I$, $CF_3(CF_2)_5I$, $(CF_3)_2CFI$, $(CF_3)_2CFCF_2I$, $(CF_3)_2CF(CF_2)_2I$ and $(CF_3)_2CF(CF_2)_3I$. Other than these alkyl groups, the method can use the unsaturated group, especially the iodides consisting of the alkenyl group, such as $CF_2{=}CF{-}CF_2I$ and $CF_3{-}CF = CFI$.

However, it is advisable that the number of carbon atoms in the fluorine-containing aliphatic iodides to be used should be at 10 or less taking into account the solubility against solvents. For the above-mentioned fluorine-containing aliphatic iodides which can be used, $CF_3(CF_2)_2CH_2CF_2I$ that is bonded with hydrogen atoms in the molecular chain is usable in addition to the above-enumerated perfluoroalkyl group or alkenyl group. In this case, it is necessary that F should be present adjacent to I. Also diiodides, i.e., $I(CF_2CF_2)_nI$ can be used. Further, other than the above $R_fI$, aromatic group substituted iodides, i.e. $C_6H_5{-}CF_2I$, $C_6H_5{-}(CF_2)_2I$, may be used.

As RCOR' to be employed for the method of the present invention, the following compounds may be used:

(1) aliphatic aldehydes including:

$HCHO$, $CH_3CHO$, $C_2H_5CHO$, $C_3H_7CHO$, $C_4H_9CHO$, $C_5H_{11}CHO$, $CH_2 = CHCHO$, $CH_3CH = CHCHO$, $(CH_3)_2C = CHCHO$, etc.;

(2) aromatic aldehyde including:

$C_6H_5CHO$, $C_6H_5CH = CHCHO$, $CH_3OC_6H_5CHO$;

(3) aliphatic ketones including:

$CH_3COCH_3$, $CH_3COC_2H_5$, $(C_4H_9)_2CO$, $CH_2 = CHCOCH_2CH_3$, $(CH_3)_2C = CHCOCH_3$;

(4) aromatic ketones including:

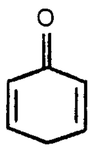 , 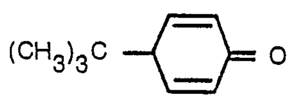 , $C_6H_5COCH_3$ , $C_6H_5COCH = CH_2$

R and R′ in RCOR′ are the similar or dissimilar atom or group, and it is permitted to use the above-stated saturated or unsaturated compounds, in addition to those introduced with a substitute group into their parts. When R and R′ are of aliphatic groups, it is advisable to limit the number of carbon atoms to 15 or less taking into considerations the solubility into solvents, and also to be capable to form rings jointly, especially cyclohexylic rings.

For the method of the present invention, it is preferable to add the above-mentioned zinc powder or $SnX_2$ powder within a range of one to three times the number of moles against $R_fI$ so as to make the reaction proceed fully and appropriately. These metallic reagents may be used independently or jointly. Where the reagents are used jointly, it is preferable to add the total amount of zinc powder and $SnX_2$ powder within a range of one to three times the number of moles against $R_fI$.

It will serve the purpose when the reaction may proceed to the full extent even under the ambient temperature and atmospheric pressure, and a range of ultrasonic waves to be obtained with a commercially available ultrasonic cleaner is employed. The tertiary amine which is added under the conditions where the halogenated tin may be of one fifths (percentage by volume) of an amount of the solvent to be used. Where solvents are used for a reaction system, it is recommended to employ aprotic solvents, such as dimethylformaldehyde, tetrahydrofuran, dimethylsulfoxide, dimethylacetamide, N-methyl-pyrrolidone, hexamethylphosphoamide, and acetonitrile.

These polar solvents have a strong dissolving action in that they have a high solvation energy against cation, and act to increase a reaction rate of anionic reagents.

For the method of the present invention, where the initiating substance itself is a liquid, it is not always necessary to use the above-mentioned solvents. And for the hydrolysis to obtain carbinol, ordinary mineral acids such as HCl and $H_2SO_4$ may be used.

The inventor has also found out that the following reactions is able to synthesize a fluorine-containing carbinol in a stabilized fashion and with a good yield, in addition to the above-described synthetic reaction.

That is, the method uses $R_fZnI$ or $R_fSnX_2I$ ($R_f$ and X are similar to those described before) (or jointly uses $R_fZ_nI$ and $R_fSnX_2I$) instead of the above-given $R_fI$. The substance is subjected to reaction with RCOR′ (R and R′ are similar to those described before) particularly under the action of ultrasonic waves (where $R_fSnX_2I$ is employed, it may be put to a reaction under the conditions where the above-given tertiaryamine is added).

Thence, where the product is subjected to hydrolysis, carbinol can be obtained as in the above-described case.

This reaction can be represented as follows for a case where $R_fZnI$ is used.

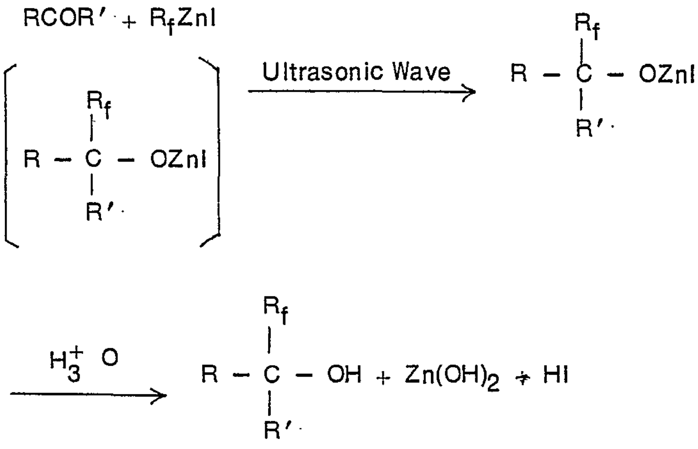


According to this reaction, it is characteristic to cause $R_fZnI$ and/or $R_fSnX_2I$ which was previously synthesized to react with carbonyl compounds. Also in this case, it is considered that, similar to the above description, the action of the ultrasonic waves or the tertiary amine may cause the closer interation between reacting molecules, and accelerate the generation of the intermediate product as mentioned before. Namely, due to the coordinate action of the ultrasonic-wave energy or the tertiary amine, it is considered that the bonding of $R_f$—Zn or $R_f$—Sn of $R_fZnI$ or $R_fSnX_2I$ in particular may be made weaker, and the interaction with RCOR' may be strengthened, thereby the interaction may proceed to the full extent.

$R_fZnI$ or $R_fSnX_2I$ itself which is used well dissolves into the given solvents, and causes the reaction to proceed in an easier manner. And this $R_fZnI$ or $R_fSnX_2I$ can be synthesized within an autoclave at a temperature at 120 to 150°C by causing the reaction of the above-stated $R_fI$ with Zn or $SnX_2$ powder (X: halogen).

In this case, it is desirable to use an amount of Zn or $SnX_2$ at one to three times the number of moles against $R_fI$. Zn and $SnX_2$ may be used simultaneously, but it is preferable that their total amount is set at one to three times the number of moles against $R_fI$.

The solvent to be used for the reaction and the conditions for reaction with carbonyl compounds are to be similar to that of the description previously given.

The fluorine-containing carbinol which is synthesized according to the method of the present invention is most useful and can be used for the applications as follows:

(1) the substance itself can be used as a solvent,

(2) the substance can be used as a synthetic intermediate for water or oil repellent agents, medicines, agricultural chemicals, dyes, surface active substance, etc.,

(3) the substance can be used as a monomer to produce fluorine-containing polymers. For instance, it forms an unsaturated bonding of a monomer by removing OH group and adjacent hydrogen atoms by means of dehydration reaction.

The method of the present invention may be based on the following reaction. That is, a fluorine-containing aliphatic iodide which is represented by the Formula, $R_fI$ (where $R_f$: fluorine-containing aliphatic group), and a halogen-substituted aliphatic unsaturated compound or a halogen-substituted aromatic compound is to be subjected to a reaction under the ultrasonic wave action at the presence of zinc powder and a palladium catalyst. Thereby, introducing the above-said $R_f$ into the above multiple bond or the benzene nucleus, an unsaturated aliphatic compound or an aromatic compound which is eleminated of the above-said halogen can be obtained.

According to this method, where the initiating substance, $R_fI$ and a halogen-substituted unsaturated aliphatic compound or a halogen-substituted aromatic compound are caused to react each other, when these substances are put under the action of ultrasonic waves by bringing them into contact with the palladium catalyst under the presence of the zinc powder, the interaction between the reacting molecules is made closer with the ultrasonic-wave energy, thereby $R_fI$ is efficiently cross-coupled with the above-stated multiple bonding position or carbon atoms of the benzene nucleus, and finally it is possible to obtain with a good yield a product which is introduced with the fluorine-containing aliphatic group ($R_f$) under the condition of dehalogenation. In this casem firstly the Zn powder reacts with $R_fI$, resulting with the stabilized $R_fZnI$, and also reacts by maintaining the full interaction with the halogen-substituted unsaturated aliphatic compound or aromatic compound which is activated under the action of the palladium-type catalyst.

Conversely, where the palladium-type catalyst is not used and the ultrasonic wave is not put into action, it is ascertained that no reaction does proceed.

It is considered that the above-described reaction may proceed as below, where the halogenated allyl is employed instead of the above-stated halogenated aliphatic unsaturated compound.

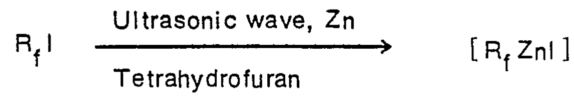


5

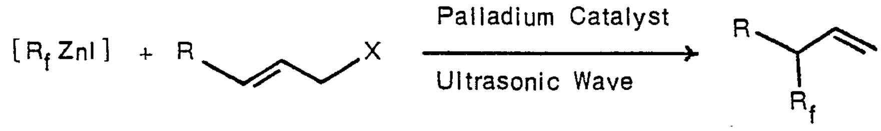


5 6 7

That is, with the reaction of $R_fI$ with Zn, the intermediate product (*5*) (i.e. perfluoroalkyl zinc iodide) is generated, and the perfluoroalkylated substance (*7*) of a halogenated allyl is produced through smooth reaction of the halogenated allyl (*6*) (where R: alkyl group; X: halogen) with the intermediate product (*5*) within the said solvent (in situ) under the contact with the palladium-type catalyst. This perfluoroalkyl group is introduced into the γ-position of the halogenated allyl (*6*) under the dehalogenation (X) condition at a probability of 95% or greater in a position-selective fashion, and thereby the desired product (*7*) bonded selectively at the γ-position with $R_f$ can be obtained with a good yield.

As the palladium- type catalysts to be used for this reaction, various types of them were tried. Namely, various palladium- type catalysts were applied at the reaction, for instance, of trifluoromethyl zinc iodide (corresponding to the above *5*) and cinnamyl bromide (corresponding to the above *6*). It was found out that the palladium acetate ($Pd(OA_c)_2$) was particularly best suited to the aspect of yield of the product as indicated on the following Table 1.

TABLE 1

| Catalyst | Yield (%) *1) |
|---|---|
| $Pd(OA_c)_2$ | 63 |
| $PdCl_2$ | 34 |
| $PdCl_2(PPh_3)_2$ *2) | 51 |
| $PdCl_2(PhCN)_2$ *3) | 23 |

*1) The measurement value by means of $^{19}F$ NMR adopting $PhCF_3$ as a comparative substance.

*2) Bis(triphenylphosphine)palladium dichloride.

*3) Bis(benzonitrile)palladium dichloride.

For this method, as a usable $R_fI$, it can include the fluorine-containing aliphatic iodides which are represented by the Formula, $CF_3(CF_2)nI$ or $(CF_3)_2(F_2)nI$. Included are: $CF_3I$, $CF_3CF_2I$, $CF_3(CF_2)_2I$, $CF_3(CF_2)_3I$, $CF_3(CF_2)_4I$, $CF_3(CF_2)_5I$, $(CF_3)_2CFI$, $(CF_3)_2CFCF_2I$, $(CF_3)_2CF(CF_2)_2I$, and $(CF_3)_2CF(CF_2)_3I$, etc.

In addition to these alkyl groups, unsaturated groups, particularly, the iodides consisting of alkenyl groups, for instance, $CF_2=CF—CF_2I$ and $CF_3—CF=CFI$ may be used. Provided that the number of carbon atoms of the fluorine-containing aliphatic iodides to be used is preferred to be at 10 or less in consideration of the solubility against solvents. Further, as the said fluorine-containing aliphatic iodides, in addition to the above-enumerated perfluoroalkyl group or alkenyl group, $CF_3(CF_2)_2CH_2CF_2I$ which is bonded with the hydrogen atoms at a part of the molecular chain may be used. In this case, it is necessary that F is present adjacent to I. Also, diiodides such as $I(CF_2CF_2)_nI$ may be used.

Further, in addition to the above-said $R_fI$, aromatic group-substituted iodides such as $C_6H_5—CF_2I$ and $C_6H_5—(CF_2)_2I$ may be used.

And as the usable halogenated aliphatic unsaturated compounds, include the above-mentioned allyl bromides, halogenated allyl compounds or their derivatives which are represented by the following Formula may be included.

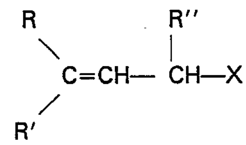

(where R, R′ and R″ indicate an hydrogen atom, alkyl group or alkenyl group, having carbon atom numbers of 10 or less than 10, aromatic group or aromatic group substituted with substituents, X: halogens such as Br, I and Cl).

Here, as R, R′ and R″, the alkyl groups such as methyl group, ethyl group, propyl group, isopropyl group, butyl group; and alkenyl groups which have unsaturated bond partially may be applied. But it is preferable to let the number of carbon atoms at 10 or less. Also, as the substituent to be introduced into the above-stated substituted aromatic group, it is necessary to employ the substituents which will not affect the development substantially in the dehalogenating reaction. As such substituent, alkyl group or alkenyl group may be adopted. In this case, the number of carbon atoms therein is preferred to be limited to 5 or less. And, other types of substituents to be introduced into these aromatic groups may be of aryl group, halogens such as Cl, nitro group, cyano group, and alkoxy group.

Also, it is possible to use the halogenated vinyl compounds or their derivatives which are represented by the following Formula, instead of the above given halogenated allyl compounds or their derivatives.

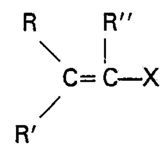

(where R, R′ and R″ are similar to those described before. X indicates the halogens such as Br, I, Cl and etc.). In this case, against the α-position (vinylic position) of the halogenated vinyl compounds or their derivatives, $R_f$ will be introduced selectively as follows under the conditions of dehalogenation (X). Tetrakis (triphenylphosphine) palladium may be used as the palladium- type catalyst.

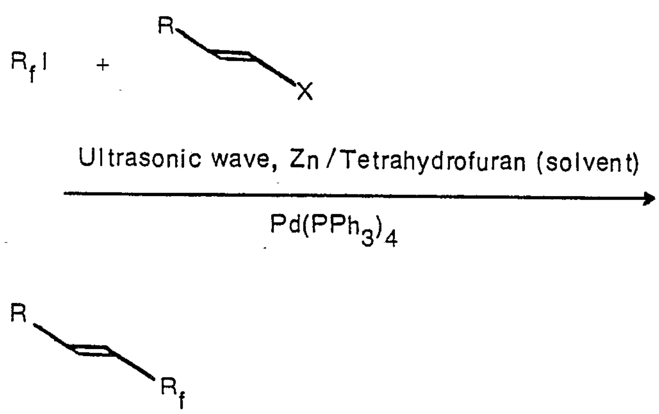


Thus, by using various $R_fI$ and derivatives of halogenated allyl or vinyl compounds, their perfluoroalkylation was made according to the method of the present invention, the results indicated on the following Table—2 were obtained.

TABLE 2

| $R_f$ | Substrate | Product *1) | Catalyst | Reaction Time (hr) | Yield (%) | Boiling Point (°C/mmHg) |
|---|---|---|---|---|---|---|
| $CF_3$ | $PhCH{=}CHCH_2Br$ | $Ph(CF_3)CHCH{=}CH_2$ *2) | $Pd(OA_c)_2$ | 1 | 63 | 81～83/25 |
| $CF_3$ | $PhCH{=}CHBr$ | $PhCH{=}CHCF_3$ | $Pd(PPh_3)_4$ | 1 | 65 | 70～72/25 |
| $CF_3$ | $4{-}CH_3C_6H_4CH$ $=CHBr$ | $4{-}CH_3C_6H_4CH$ $=CHCF_3$ | $Pd(PPh_3)_4$ | 1 | 67 | 59～60 *3) |
| $CF_3$ | $4{-}CH_3C_6H_4CH$ $=CHCH_2Br$ | $4{-}CH_3C_6H_4(CF_3)CHCH$ $=CH_2$ | $Pd(OA_c)_2$ | 1 | 67 | 90～92/26 |
| $n\text{-}C_3F_7$ | $PhCH$ $=CHCH_2Br$ | $Ph(C_3F_7^n)CHCH{=}CH_2$ *2) | $Pd(OA_c)_2$ | 0.5 | 71 | 81～83/21 |
| $n\text{-}C_3F_7$ | $PhCH$ $=CHBr$ | $PhCH{=}CHC_3F_7$ *2) | $Pd(PPh_3)_4$ | 1 | 66 | 78～80/24 |
| $i\text{-}C_3F_7$ | $PhCH$ $=CHCH_2Br$ | $Ph(C_3H_7^i)CHCH{=}CH_2$ *2) | $Pd(OA_c)_2$ | 0.5 | 78 | 80～82/21 |
| $i\text{-}C_3F_7$ | $PhCH$ $=CHBr$ | $PhCH{=}CHC_3F_7^i$ *2) | $Pd(PPh_3)_4$ | 1 | 72 | 75～77/24 |
| $n\text{-}C_4F_9$ | $CH_3CH$ $=CHCH_2Br$ | $CH_3(C_4F_9^n)CHCH{=}CH_2$ *2) | $Pd(OA_c)_2$ | 0.5 | 68 | 81～84 *3) |
| $n\text{-}C_4F_9$ | $PhCH$ $=CHBr$ | $PhCH{=}CHC_4F_9^n$ *2) | $Pd(PPh_3)_4$ | 1 | 62 | 76～78/23 |

*1): The structure of products was identified with IR, NMR and mass spectrometry.
*2): This is a new compound, and its elementary analysis result has fully coincided with the calculated value. (C, H, N: ± 0.4).
*3): Indicates the melting point (°C).

Among the above-given new compounds (*2), the identified data (NMR) of the allyl compound are specifically given below.

| | | |
|---|---|---|
| $Ph(CF_3)CHCH=CH_2$: | | |
| | $^1H$ NMR: | τ 5.2, 5.0, 5.9 ($CH=CH_2$) |
| | | 4.7 ($CH(CF_3)$) |
| | | 7.5 (Ar—H) |
| | $^{19}F$ NMR: | −22 ($CF_3COOH$ is determined to be the external standard) |
| $Ph(C_3F_7^n)CHCH=CH_2$: | | |
| | $^1H$ NMR: | τ 5.8, 5.2, 5.1 ($CH=CH_2$) |
| | | 4.6 (CH—) |
| | | 7.4 (Ar—H) |
| | $^{19}F$ NMR: | −9.0 ($CF_2$) |
| | | 1.5 ($CF_3$) |
| | | 41.0 ($CF_2$) |
| $Ph(C_3F_7^i)CHCH=CH_2$: | | |
| | $^1H$ NMR: | τ 6.1, 5.4, 5.2 ($CH=CH_2$) |
| | | 4.8 (CH—) |
| | | 7.5 (Ar—H) |
| | $^{19}F$ NMR: | −7.0 ($CF_3$) |
| | | 102 (CF) |
| $CH_3(C_4F_9^n))CHCH=CH_2$: | | |
| | $^1H$ NMR: | τ 6.5, 6.2, 5.0 ($CH=CH_2$) |
| | | 1.6 ($CH_3$) |
| | | 4.7 (CH—) |
| | $^{19}F$ NMR: | −4.0, 36.0, 40.0 ($CF_2$) |
| | | 3.1 ($CF_3$) |

Further, instead of the above-described halogenated aliphatic unsaturated compounds, the halogenated aryl compounds or their derivatives as the halogen-substituted aromatic compounds which are represented by the following Formula may be used.

In this case, with the carbon atoms which are bonded with the halogen, the cross coupling is generated.

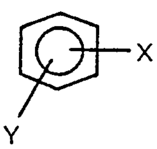

(where X indicates halogens such as Br, I and Cl; Y indicates at least one type of the substituent or hydrogen atom, which is selected from the groups consisting of alkyl group or alkenyl group having carbon atom numbers of 5 or less, or cyano group, nitro group, ester group and aryl group).

Here, as Y, the alkyl group or alkenyl group such as methyl group and ethyl group can be applied, but it is preferable to limit the number of the carbon atoms to 5 or less. Also, the above-given substituent (Y) may be introduced with the number of 1 to 3 (or at one to three locations) against the benzene nucleus. The Ullmann-type reaction is known as a method to introduce the perfluoroalkyl group into those halogenated aromatic compounds, but the handling is extremely difficult due to difficulty in the preparation of copper powder to be used, and that the reaction is significantly affected in proportion to the amount of the copper powder used. Conversely, the method of the present invention is very easy in handling, and can introduce $R_f$ into the aromatic compounds with a higher yield. As the palladium- type catalyst, when $PdCl_2$ or $Pd(PPh_3)_2Cl_2$ is employed, it is ascertained that a sufficient catalytic function is presented. When the palladium black was employed instead of $PdCl_2$, and the benzene iodide and the heptafluoro (1-methylethyl)zinc iodide let reacted with the heptafluoro (1-methyl ethyl)benzene was obtained with a yield by 53%. This fact suggests that Pd(0) which is thought to be generated from $PdCl_2$ or $Pd(PPh_3)_2Cl_2$ at the action of ultrasonic waves under the presence of Zn powder is acting as a catalyst actually. It is considered that this Pd (0) forms the following reduction-oxidation cycle, and following this cycle, $R_f$ is effectively introduced into the benzene nucleus.

$$R_fI + ArI \xrightarrow[Pd(PPh_3)_2Cl_2]{\text{Ultrasonic waves, Zn/Tetrahydrofuran}} R_f\text{–}Ar$$

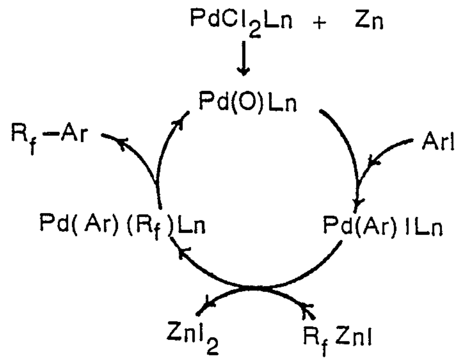

Various reactions were caused on the above-given halogenated aromatic compounds or their derivatives following the method of the present invention, and obtained the results of which the Table-3 below shows.

TABLE 3

| $R_f$ | ArI | Product *1) | Yield (%) | Melting Point (°C) |
|---|---|---|---|---|
| $CF_3$ | PhI | $PhCF_3$ | 82 | 101~103 |
| $n\text{-}C_3F_7$ | PhI | $PhC_3F_7^n$ | 78 | 127~130 |
| $n\text{-}C_4F_9$ | PhI | $PhC_4F_9^n$ | 81 | 82~84/48 *2) |
| $i\text{-}C_3F_7$ | PhI | $FhC_3F_7^i$ | 87 | 124~126 |
| $i\text{-}C_3F_7$ | $4\text{–}CH_3C_6H_4I$ | $4\text{–}CH_3C_6H_4C_3F_7^i$ | 81 | 146~148 |
| $i\text{-}C_3F_7$ | $3\text{–}CH_3C_6H_4I$ | $3\text{–}CH_3C_6H_4C_3F_7^i$ | 68 | 145~147 |
| $i\text{-}C_3F_7$ | $2\text{–}CH_3C_6H_4I$ | $2\text{–}CH_3C_6H_4C_3F_7^i$ | 59 | 140~143 |

*1) : The structure of the product was identified by 1R, NMR, and mass spectrography analysis.

*2) : Indicates a boiling point (°C/mmHg).

With this method, it is preferable to let an amount of zinc powder to be added in the course of reaction being limited within a range of 1 to 3 times (molar ratio) that of $R_fI$. As the initiating substances to be used are stabilized respectively, and the reaction can be caused within a single reactor under the condition of the ambient temperature and atmospheric pressure, the method is most practicable because of an easier handling.

It is enough to use ultrasonic waves obtainable from a commercially available ultrasonic-wave cleaner in order to let act under the presence of zinc powder and palladium-system catalyst. Thus the handling is made easier.

Where solvents are used for the reaction according to the method of this invention, it is preferable to employ aprotic polar solvents such as dimethylformamide, tetrahydrofuran, dimethylsulfoxide, dimethylacetamide, N-methylpyrrolidone, hexamethylphosphoamide, acetonitrile and so on. As these polar

catalysts possess a greater solvation energy against cation, they present a stronger dissolving action, and accelerate the reaction rate of anionic reagents. Further, according to the method of the present invention, where the initiating substance itself is of liquid, it is not always necessary to use the above-described solvents.

The present inventor has found out that, apart from the above-stated synthetic reaction, the desired fluorine-containing aliphatic unsaturated compound or fluorine-containing aromatic compounds can be synthesized with the following reaction in a stabilized fashion and with a good yield.

That is, as an initiating substance, a fluorine-containing aliphatic zinc iodide represented by the following formula instead of the above-given $R_fI$ is employed.

$$R_fZnI$$

(where $R_f$ is similar to the above-given one).

This $R_fZnI$ is let reacted under the ultrasonic wave action and under the presence of a halogen-substituted aliphatic unsaturated compound or a halogen-substituted aromatic compound and a palladium-type catalyst.

Thereby, the above-mentioned $R_f$ is introduced into the above-said multiple bond or benzene nucleus, and it is possible to obtain an aliphatic unsaturated compound or aromatic compound which is eliminated of the above-stated halogen. This reaction is characterized by the employment of the $R_fZnI$ itself which is considered to be an intermediate product at the respective reaction described as above as an initiating substance.

This is represented, for instance, as follows.

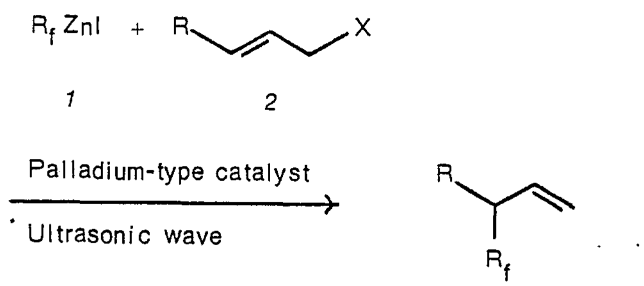


1 2 3

Thence, the product similar to that of the above, for instance, a fluorine-containing allyl compound derivative (*3*) can be obtained. The reaction conditions for palladium-system catalysts, ultrasonic waves, solvents and etc. may be similar to that of above-described one.

According to this reaction it is characteristic to use the pre-synthesized $R_fZnI$. Even in this case, it is thought that, similar to the above description, the action of ultrasonic waves causes a closer interaction between the reacting molecules, and with the catalystic action of a palladium-type catalyst, the generation of a desired product may be accelerated. That is, it is presumed that, particularly the bonding of $R_f$—Zn of $R_fZnI$ is made weaker due to the ultrasonic wave energy, and also the interaction with the reacting substance is increased, and the reaction between both of them may proceed to the full extent.

The $R_fZnI$ itself well dissolves into the solvent, thereby the reaction proceeds in a ready manner. Also, the $R_fZnI$ can be synthesized by causing the above-said $R_fI$ and Zn powder to react each other within an autoclave, for instance, at a temperature range, 120 to 150°C. In this case, it is advisable to use an amount of Zn at one to three times (the number of moles) that of $R_fI$.

The above-described fluorine-containing compounds which are synthesized by means of the method of the present invention are useful as the synthetic intermediate for water-or oil-repellent agents, medicines and agricultural chemicals, and surface active agents, or the monomers to produce fluorine-containing polymers.

With the method of the present invention, fluorine-containing allyl derivatives represented by the following formula can be obtained.

Formula:

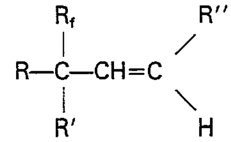

(where $R_f$ indicates the fluorine-containing aliphatic group preferably with the number of carbon atoms of 10 or less; R, R' and R'' indicate the hydrogen atom, the alkyl group or alkenyl group preferably having

carbon atoms of 10 or less, or aromatic group, or aromatic group substituted by the substituent).

As the $R_f$ of this fluorine-containing allyl derivatives represented by the above Formula, the fluorine-containing aliphatic groups represented by the Formula: $CF_3(CF_2)n—$ or $(CF_3)_2CF(CF_2)n—$ can be mentioned. Included are $CF_3—$, $CF_3CF_2—$, $CF_3(CF_2)_2—$, $CF_3(CF_2)_3—$, $CF_3(CF_2)_4—$, $CF_3(CF_2)_5—$, $(CF_3)_2CF—$, $(CF_3)_2CFCF_2$, $(CF_3)_2(CF_2)_2—$, $(CF_3)_2CF(CF_2)_2—$, and so on.

Apart from these alkyl groups, it is possible to use the unsaturated group, particularly the alkenyl group, for instance, $CF_2 = CF—CF_2—$, $CF_3—CF{=}CF—$, and the like. However, it is preferable to limit the number of carbon atoms of the fluorine-containing aliphatic group to be used to 10 or less taking into account the solubility against solvents. And, as the above-described fluorine-containing aliphatic group, it is possible to use not only the above-enumerated perfluoroalkyl group or alkenyl group, but also, for instance, $CF_3(CF_2)_2CF_2CF_2—$ which is bonded with the hydrogen atoms at a part of the molecular chain. In addition, as the above-given $R_f$, other than the above, the aromatic group substituted aliphatic groups, for instances, $C_6H_5—CF_2—$, $C_6H_5—(CF_2)_2—$ and the like may be used.

Also, as the above given R, R' and R'', apart from the hydrogen atoms, the alkyl groups such as methyl group, ethyl group, propyl group, isopropyl group, butyl group or alkenyl groups containing partially unsaturated bond may be applied, but it is preferable to limit the number of carbon atoms to 10 or less. And, where the aromatic group substituted with the substituent is applied, it is necessary to use the substituent which will not substantially affect the proceeding of dehalogenating reaction. As those substituents, the alkyl group or alkenyl group may be used, but it is advisable to limit the number of the carbon atoms to 5 or less.

Also, as the other substituents, other than the above-described alkyl group or alkenyl group, the aryl group, halogens like Cl, nitro group, cyano group and alkoxy group containing 5 or less number of the carbon atoms may be counted.

Further, the present invention proposes another method, in producing the desired fluoroketone groups, which is characterized with a process to generate the metallic halogenated ketone represented by the Formula:

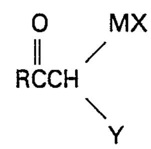

(where R represents the aliphatic hydrocarbon oxy group, aliphatic hydrocarbon group or fluoro aliphatic hydrocarbon, and includes those forming rings between the carbon atoms being bonded with X; X : halogen; Y : hydrogen or fluorine atom; M:zinc or magnesium), with the metal (M) consisting of zinc or magnesium, by causing the reaction with the halogenated ketone which is represented by the Formula:

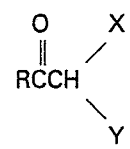

(where R, X and Y are similar to the above-given description); and with a process to generate the fluorodiketone which is represented by the Formula:

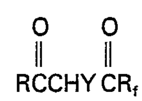

(where R and Y are similar to the previous description, and $R_f$ is fluoro-aliphatic hydrocarbon group and includes those containing ether bonds within the chain) by using the above-given metallic halogenated ketone to react with the fluoroester which is represented by the Formula:

$$R_fCOOR'$$

(where $R_f$ is as described above, and R' is an aliphatic hydrocarbon group or aromatic hydrocarbon group) under the action of ultrasonic waves.

That is, this method took advantage of the strong, electrophilic property of $R_f$ under the presence of fluorine in particular within the structure of the above-given fluoroester, and by making the interaction between the fluoroester and the above-given metal halogenated ketone by means of the energy of

ultrasonic waves (it is enough to use a commercially available ultrasonic cleaner), a full and easier reaction between both of them were attained. Thereby, it became possible to fluorinate the objects with the use of various fluoroesters, and the production of them became able to be processed with a higher yield under the milder conditions. In addition, it was found out that the reaction could proceed satisfactorily by using the commercially available zinc and magnesium without refining them, contrary to the case of the conventional Reformatsky reaction.

The method of the present invention can cause the above-given halogenated ketone to react with the fluoroester subjecting them to the action of ultrasonic waves under the presence of zinc or magnesium. It is therefore possible to obtain the desired compound by putting all the reaction reagents into a reactor from the start with the action of the ultrasonic waves. Conversely, it also is able to obtain the desired product, firstly by generating the above-said metal halogenated ketone with the reaction of the above-described ketone with zinc or magnesium under the action of ultrasonic waves, then by letting that ketone to react with the above fluoroester under the action of ultrasonic waves. In this case, the generation of the above-described metallic halogenated ketone will not always require the action of ultrasonic waves, and it is possible to obtain the metallic halogenated ketone by causing the reaction of the halogenated ketone with zinc or magnesium under heating.

At the method of the present invention, it is preferable to let the above-stated reaction proceed within the aprotic polar solvents so as to accelerate a reaction rate. As the polar solvent, it is possible to use the tetrahydrofuran, dimethylformamide, acetonitrile, dimethylsulfoxide, dimethylacetamide, N-methyl-pyrolidone, hexamethylphosphoamide or mixtures of two or more of the above substances.

Secondly, for the reaction reagents to be used for the method of the present invention, it is possible to apply the aliphatic hydrocarbon-oxy group (alkoxy group such as $—OCH_3$, $—OC_2H_5$, $—OC_3H_7$, $—OC_4H_9$), aliphatic hydrocarbon group (alkyl group such as $—CH_3$, $—C_2H_5$, $—C_3H_7$, $—C_4H_9$ or alkenyl group with the number of the carbon atom similar to the former), or fluoro aliphatic hydrocarbon group (fluoroalkyl group such as $—CF_3$, $—C_2F_2$, $—C_3F_7$, $—C_4F_9$ or fluoroalkenyl group with the number of carbon atom similar to the above), all of which R is to be with the number of carbon atom at 10 or less. In addition, it is sufficient that this R may be the one which forms rings between the carbon atoms which are bonded with the above-described Y, as in the case of d-3-halogenated camphor. Also, it is preferable that X contained in the halogenated ketone is to be of the bromine atom or iodine atom.

Further, at the above-described fluoroester, $R_f$ which becomes the source to introduce fluorine into the subject compound may consist of fluoroalkyl group or alkenyl group which contains the carbon atoms in the number of 15 or less. Included are: fluoroalkyl group such as $—CF_3$, $—C_2F_2$, $—C_3F_7$, $—C_4F_9$ or $—C_5F_{11}$, or fluoroalkenyl containing the similar number of the carbon atoms, also included are those fluoroalkenyl groups or alkenyl groups which are partially substituted and introduced with other halogens (i.e. Cl).

Also, this $R_f$ may be the fluoroalkylether group which is represented by the following Formula, and which can form an optical isomer containing the asymmetric carbon, and forms a shift reagent being superior in NMR analysis (nuclear magnetic resonance analysis)

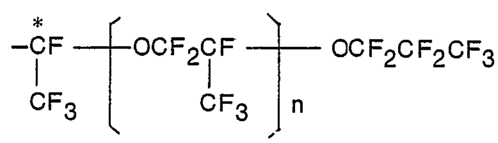

(where $n = 0 \sim 3$; $C^* =$ asymmetric carbon)

And also, the R′ contained in this fluoroester may consist of the above-given alkyl group or alkenyl group which contains the carbon atoms in the number of 10 or less.

Here, the above-stated method is described in a more detailed fashion.

First, the Reformatsky-type reagent (*9*) is generated by causing the brominated ester (*8*) to react with zinc powder under the action of ultrasonic waves, then by letting this reagent (*9*) reacted with the fluoroalkylester (*10*) under the action of ultrasonic waves, the desired β-fluoroalkyl-β-ketocarboxylic acid ester (*11*) can be obtained.

$$BrCHYCOOC_2H_5 + Zn$$

8

$$\xrightarrow[\text{Mixed solvents}]{\text{Ultrasonic wave}} BrZnCHYCOOC_2H_5$$

9

(Tetrahydrofuran: dimethylformamide = 2:1)

$$BrZnCHYCOOC_2H_5 + R_fCOOR'$$

9 10

$$\xrightarrow{\text{Ultrasonic wave}} C_2H_5O\overset{O}{\overset{\|}{C}}CHY\overset{O}{\overset{\|}{C}}R_f$$

11

With this reaction, a $R_fCO$ group could be selectively introduced into the object *11* within the similar reactor under mild conditions, and resulted with a very good yield as shown on the following Table 4.

TABLE 4

| Y in *8* | $R_fCOOR'$ *10* | Products *11* | Yield (%) |
|---|---|---|---|
| H | $CF_3COOC_2H_5$ | $C_2H_5OCOCH_2COCF_3$ | 83 |
| H | $C_2F_5COOC_2H_5$ | $C_2H_5OCOCH_2COC_2F_5$ | 86 |
| H | $ClCF_2COOCH_3$ | $C_2H_5OCOCH_2COCF_2Cl$ | 65 |
| F | $CF_3COOC_2H_5$ | $C_2H_5OCOCHFCOCF_3$ | 78 |
| F | $C_2F_5COOC_2H_5$ | $C_2H_5OCOCHFCOC_2F_5$ | 76 |
| F | $ClCF_2COOCH_3$ | $C_2H_5OCOCHFCOCF_2Cl$ | 52 |

However, when the ordinary hydrocarbon-system RCOOR' was used instead of $R_fCOOR'$ at the above-described reaction, the reaction did not proceed even under the irradiation of ultrasonic waves, and the desired ketocarboxylic acid ester could not be obtained. This shows that $R_f$ of the $R_fCOOR'$ used for the present invention has strong electrophilic property, and that this fact contributes to fully proceed the nucleophilic reaction with the Reformatsky type reagent.

In the meantime, at the above-described reaction, even when magnesium instead of zinc was used to let react with $R_fCOOR'$ according to the Grignard method, it was found out that the required ketocarboxylic acid ester (*11*) could be obtained. This fact also is considered that the reaction may proceed with a high yield due to the above-given properties of $R_f$ and the action of the ultrasonic wave energy.

Also, at the above reaction, it is possible to use, for the halogenated ketone (*8*) to produce the reagent (*9*), the substance represented by the Formula:

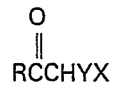

can be used.

This halogenated ketone may be the one which introduces fluorine into the object. In this case, it is enough to substitute R with the fluoroalkyl group ($R_f$). However, when the bond between R—C of ketone is split and R is eliminated in the course of the reaction, and if fluorine (F) is adopted for the above-described Y, even when R is not present in the object, fluorine as Y can be introduced, and the physiological activity can be maintained.

Further, as this halogenated ketone, when the brominated acetone ($BrCHYCOCH_3$) of which R consists of the alkyl group is used instead of the above-described brominated ester (*8*), the product (*11*) can be obtained with a good yield.

When the brominated fluoroacetone (i.e.

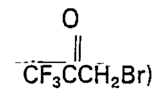

(which substitutes this R with the fluoroalkyl group ($R_f$) is used, and this substance is let reacted with the fluoroester (i.e.

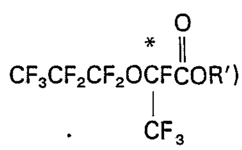

according to the following reaction Formula, a superior shift reagent (*12*) can be obtained.

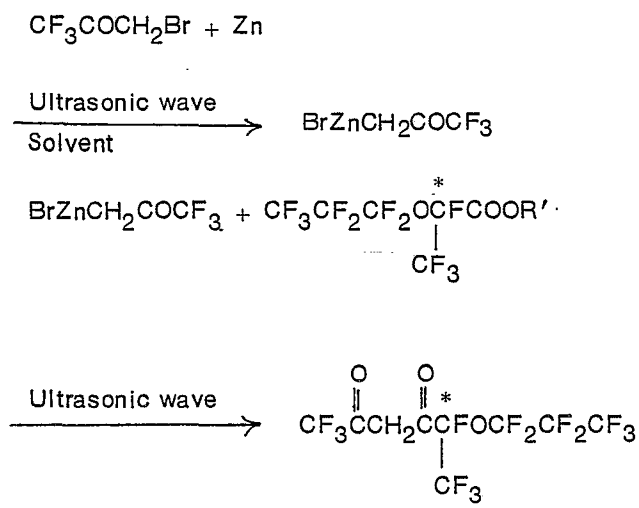


*12*

As this shift reagent has the ether bond which is bonded with the oxygen atoms between the asymmetric carbon and perfluoropropyl group ($—CF_2CF_2CF_3$), the bond is stabilized by oxygen, and even when the nucleophilic reagent may act,

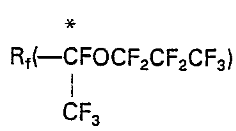

will not be eliminated.

Also, it is possible to select various $R_f$'s as above, particularly as the $R_f$ of the above-described fluoroester ($R_fCOOR'$), the above-stated

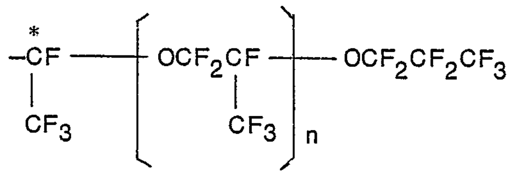

(where n = 0~3) may be applied.

Then, description is made on an example which introduced the $R_fCO$ group into d-camphor as a shift reagent for NMR.

According to the similar process described as above, d-3-bromcamphor (*13*) was let reacted with perfluoroalkylester ($R_fCOOR'$) (*10*) being irradiated with the ultrasonic waves according to the following Formula under the presence of zinc powder, and obtained the desired shift reagent (*14*) with ease and a high yield.

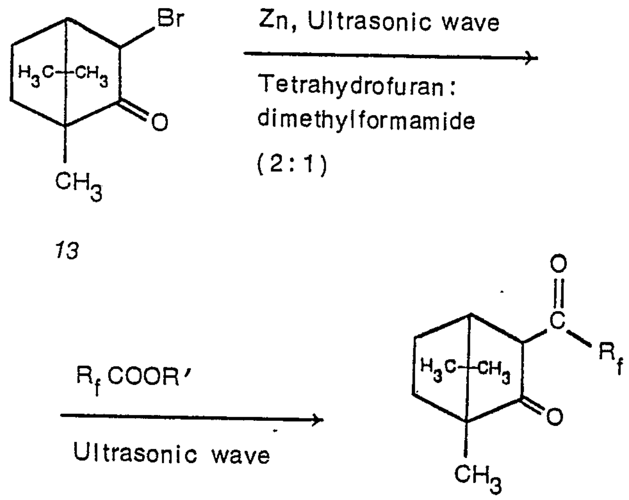


*13* *14*

This d-camphor derivative (*14*) is excellent as a shift reagent, and was obtained with a high yield as shown in the following Table 5.

TABLE 5

| $R_fCOOR'$ | Product *14* | *Yield* (%) |
|---|---|---|
| $CF_3COOC_2H_5$ | 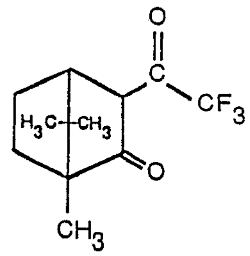 | 86 |
| $C_2F_5COOC_2H_5$ | 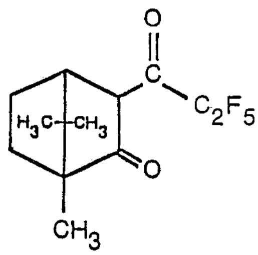 | 84 |
| $C_3F_7COOC_2H_5$ | 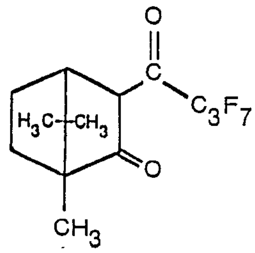 | 76 |
| $ClCF_2COOCH_3$ | 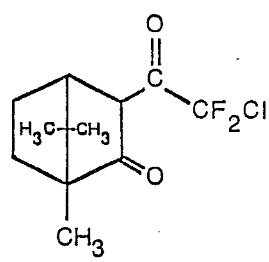 | 61 |
| 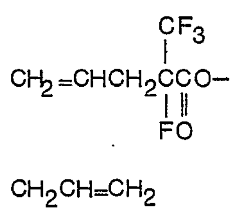 | 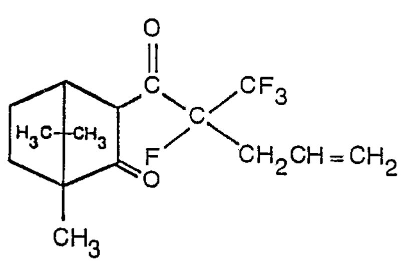 | 51 |

As described above, the method of this invention utilizes the chemical properties of the fluoroaliphatic hydrocarbon group ($R_f$) and the action of ultrasonic wave in combination, so that several remarkable effects which can not be expected by the conventional methods can be obtained. Particularly, as the reaction can proceed under the mild condition without being accompanied by severe reaction, the handling is easy, and various fluoroesters ($R_fCOOR'$) to be used can be selected without any restriction. Also, even when the commercial zinc powder or magnesium powder is used without refining, it presents a full activity, and further, the commercially available solvents may be well used only drying them by means of a molecular sieve, for instance, without refining.

The fluoro-β-diketone which can be obtained by the method of this invention are characterized being represented by the following Formula:

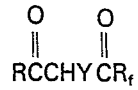

(where R represents a fluorohydrocarbon group, and fluorohydrocarbon group or hydrocarbon group which have ether bonds in their chain such as

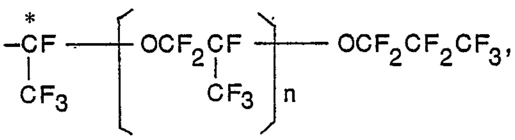

including those forming rings between the carbon atoms which are bonded with Y, Y is hydrogen atom or fluorine atom, $R_f$ represents a fluorohydro-carbon group, and includes those having ether bonds in their chains (i.e. the ether bond similar to that of the above-given R.)).

The compounds among these fluoro-β-diketones in which the asymmetric carbon C is present are effective to form a chiral shift reagent such as europium chelates. That is, due to the fluorine atoms,

1) the electrophilic property is strengthened, and the chelate is activated, resulting in the increase of the Lewis acidity as the shift reagent, thereby an excellent shift effect is obtained even at a lower concentration.

2) As the lesser presence of the hydrogen atoms which overlap the substrate, the overlapping with the signal of the substrate becomes decreased.

3) The solubility against the organic solvents is increased.

4) when the R in the above-given Formula is

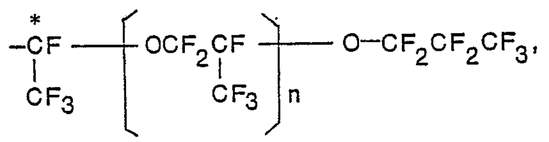

as it becomes a symmetric structure from the viewpoint of the molecular structure, the shift reagent is stabilized due to the steric effect, and it is made possible to have two asymmetric centers (carbon).

In addition, these fluoro-β-diketone groups are expected to present the above-described action as the fluorine compounds due to the presence of fluorine.

Further, structurally, when R and/or $R_f$ in the above Formula is of a fluoroalkyl group which is represented by the Formula:

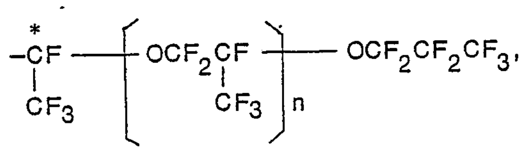

(where $n = 0 \sim 3$),

the ether bond, due to 0 between fluoroalkyl groups, contributes to stabilize that structure.

That is, when there is no such ether bond, and the fluoroalkyl group is directly bonded to the above-given

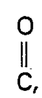

and

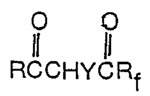

(for $R_f$: 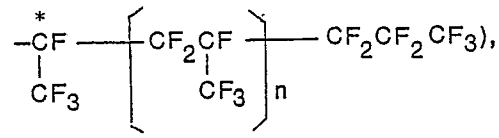

the $R_f$ acts as a pseudo-halogen, and is eliminated easily due to the action of the nucleophilic reagent onto

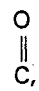

but because of the above-stated ether bond, such chemical change is prevented, and enables to maintain the fluoroalkyl group within the molecule in a stabilized fashion.

At the above Formula, the R may be of a fluoroalkyl group or alkyl group comprising the carbon atoms with the number of 7 or less, such as $—CF_3$, $—C_3F_7$, $—C_4F_9$, or $—CH_3$, $—C_2H_5$, $—C_3H_7$, $—C_4H_9$ respectively. And also this R may be of those forming the rings between the carbon atoms which are bonded with the above-given Y as in the case of d-camphor. These fluoro corpora of d-camphor also are useful as a shift reagent for NMR.

Here, the above-described fluoro-β-diketones are exemplified in a concrete manner according to the production processes which are different from the method of the present invention.

The perfluoro carbonic acid, for instance, perfluoro-2-propoxypropionic acid fluoride

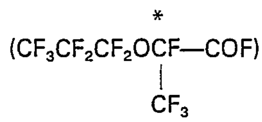

is obtained by means of the dimerization reaction of hexafluoro-1, 2-epoxypropane. This perfluoro carbonic acid fluoride (*15*) generates a diasteramer mixture by condensing together with (−)-α-phenylethylamine (*16*) according to the following Formula. Thence, the product which is obtained by separating the above-said mixture by means of column gas chromatography is hydrolyzed, and optically resolved to optically active perfluoro carboxylic acid (15′)

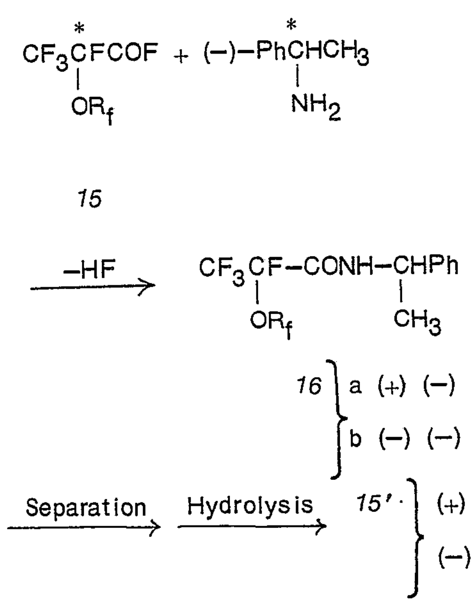


15

16 a (+) (−)
b (−) (−)

15′ (+)
(−)

Further, at this perfluoro carboxylic acid (15′), when a perfluoroisopropyl group is applied as the above-described $R_f$, it is known that the *15′* can be optically resolved in an extremely effective fashion from the mixture 16′. Among them, it is advisable to obtain perfluoro-2-isopropoxypropionic acid through fluoride (17) which is synthesized according to the following Formula.

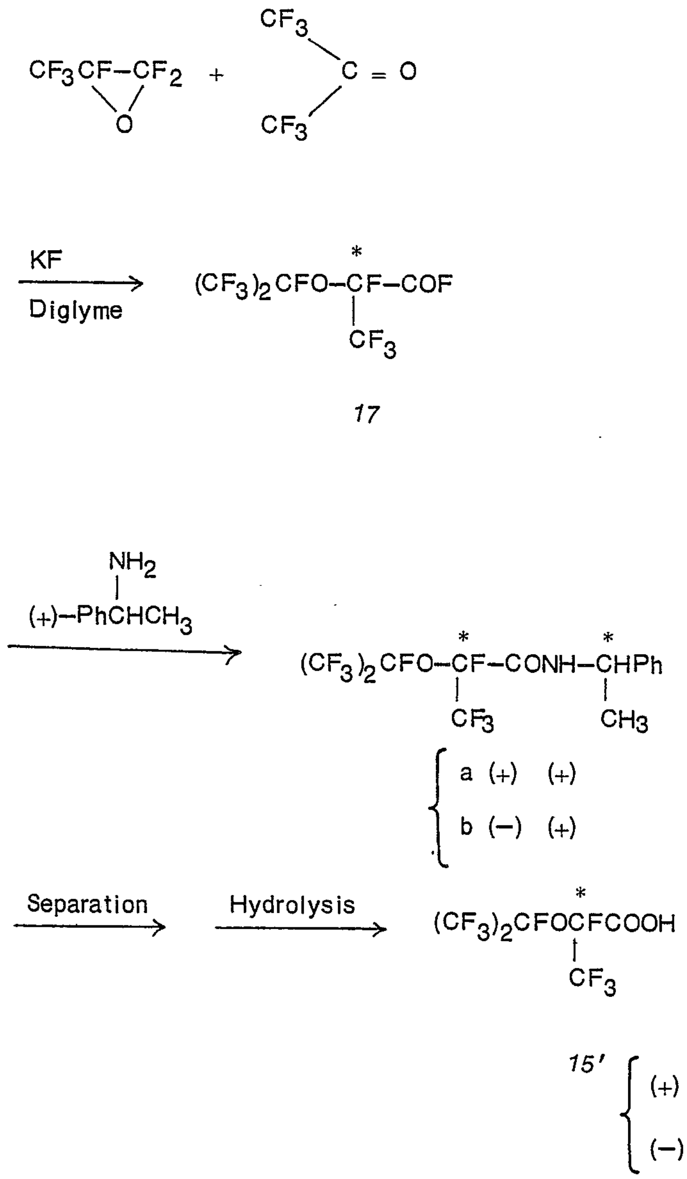


17

15'

On the other hand, the enolate anion (*19*) which is obtained from the tert-butyl acetate (*18*) in accordance with the following Formula is known to be a useful reagent to obtain the corresponding β-diketone from carboxylic acid chloride.

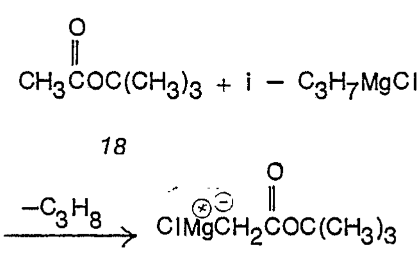


18

19

Thence, (+)-perfluoro-2-propoxypropionic acid (*15*), for instance, among the above-given perfluoro carboxylic acid which was optically resolved, was led to its acid chloride;

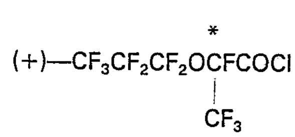

(*20*)

with phosphorus pentachloride, which was caused to react with the following Formula; thereby the desired chiral β-diketone-di(perfluoro-2-propoxypropionyl)-methane (*21*) was synthesized. It is possible to obtain this diketone (*21*) with a good yield (i.e. 90%).

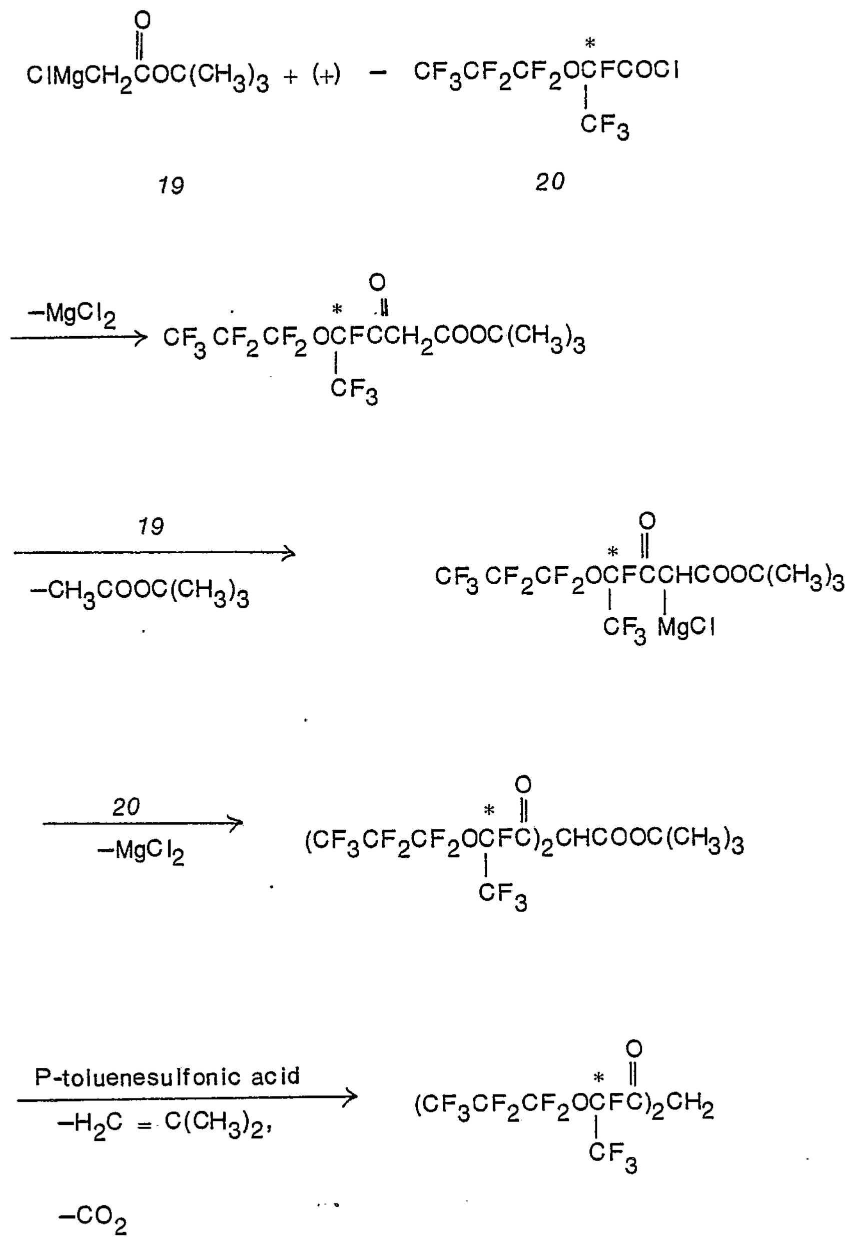


*19* *20* *21*

(+)-β-diketone-di(perfluoro-2-propoxypropionyl)-methane (*21*) thus obtained was further led to the undergiven corresponding (+)-europium chelate (*22*) in accordance with the established method.

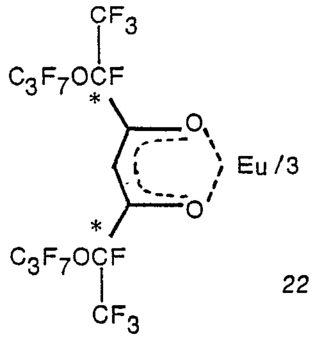

22

The following Table 6 shows the results examined on 1H NMR of several substances by adding this europium chelate (*22*).

TABLE 6

| Substrate | Mole ratio (22/Substrate) | Signal Position | ΔΔδ (ppm) |
|---|---|---|---|
| $PhCH(CH_3)NH_2$ | 0.11 | $CH_3$ | 0.20 |
| $PhCH(CH_3)OH$ | 0.13 | $CH_3$ | 0.08 |
| $CH_3CH_2\overset{*}{C}H(CH_3)COCH_3$ | 0.16 | $\overset{*}{C}H_3$ | 0.08 |
| $PhSOCH_3$ | 0.10 | $CH_3$ | 0.08 |

According to these results, despite the europium chelate (*22*) as a shift reagent is extremely low in its concentration, a satisfactory ΔΔδ value (a magnitude of shift difference between enantiomers) is obtained, thereby it can be ascertained that the europium chelate (*22*) presents a superior shift effect. Conversely, where the publicly known tris-(3-heptafluorobutyryl-d-campharate) europium (III) is added as a shift reagent, and where α-phenylethylamine is adopted as a substrate, ΔΔδ = 0.17 ppm can be obtained only when a mole ratio between a shift reagent and a substrate is raised to a high concentration at 0.40 to 0.60. This fact means that the shift reagent which uses fluoro-β-diketone is suffice to be of a concentration less than the public known only by 1/4 to 1/6, and fully indicates a superiority of this invention.

Also, as the above-given shift reagent (*22*) contained no hydrogen atoms which will overlap the substrate, it was found out that a very neat $^1$H NMR spectrum could be obtained. In addition, this shift reagent can dissolve into nonpolar organic solvents like carbon tetrachloride, specifically, it dissolves unlimitedly into the fluorine-system solvent such as 1,1,2-trichlorotrifluoroethane, and can be maintained in a stabilized fashion within the given solution. Thus, where the NMR analysis is conducted, the above-described shift reagent can be added in the form of 1,1,2-trichloro-trifluoroethane solution by means of a microsyringe and the like.

Further, the above-described europium chelate (*22*) has not only the above-mentioned conspicuous advantages, but also a pair of perfluoroproxyethyl groups

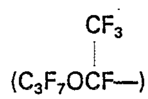

which have asymmetric carbon atoms respectively are positioned symmetrically. Thereby, due to its steric effect, the molecular structure is kept stabilized, and also as it has two asymmetric centers, it presents a superior shift effect. This europium chelate generally acts to shift the spectrum toward the lower magnetic field side as a shift reagent during the NMR analysis. Contrary to that intrinsic characteristic, it is possible to form praseodymium chelate which places Pr (praseodymium) as the central atom of chelate instead of the above-given Eu so as to shift the spectrum toward the higher magnetic field side. In addition, the above-described substance (*21*) can be obtained with a good yield by means of the method of the present

invention. Also, the above-given shift reagent (*14*) forms the chiral reagent as shown by the following Formula:

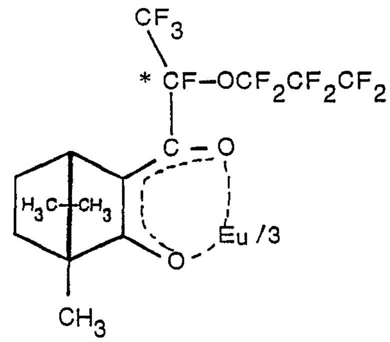

Further, here are given more detailed description on the examples which were experimented for the present invention. The under-described examples, however, will not set limits to the present invention, and that they may be deformed being based on its technical concept.

Example 1

Commercially available zinc powder (0.02 gr atom), trifluoromethyl iodide 2.15 gr (11 mmol), benzaldehyde 1.06 gr (10 mmol), and dimethylformamide 25 ml which is dried by means of a molecular sieve, were put into an eggplant-formed flask (capacity: 50 ml), and they were caused to react for 30 minutes within a water bath under the action of ultrasonic waves by means of a commercially available ultrasonic cleaner (35W, 32kHz).

Upon completion of the reaction, and the solution was hydrolyzed by adding 100 ml of 2% hydrochloric acid, thereby an oil layer was extracted by means of diethylether.

Then, after drying this liquid thus extracted with magnesium sulfate, the solvent was removed by means of fractional distillation. Through vacuum distillation of the residue, phenyltrifluoromethylcarbiol

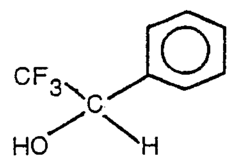

was obtained with a yield by 72% (1.27 gr). The boiling point of this carbinol was ascertained to be at 105~108°C/16 mmHg.

Example 2

At the Example 1, by using tetrahydrofuran (25 ml) as the solvent, and let the solution undergo the reaction under the conditions identical to the experiment 1, phenyltrifluoromethycarbinol was obtained with a yield by 56% (0.99 gr).

Example 3

Commercially available zinc powder 1.30 gr (0.02 gr atom), trifluoromethyl iodide 2.15 gr (11 mmol), acetophenone 1.20 gr (10 mmol), and dimethylformamide 25 ml were put into an eggplant-formed flask (capacity: 50 ml) respectively, and let them to react for one hour within a water bath under the action of ultrasonic waves by means of a commercially available ultrasonic cleaner (35 W, 32 KHz). Upon completion of the reaction, 2% hydrochloric acid (100 ml) was added, and an oil layer was extracted by means of diethylether.

After drying this extracted liquid with magnesium sulfate, the solvent was removed through fractional distillation. Through vacuum distillation of the residue, methylphenyltrifluoromethylcarbinol

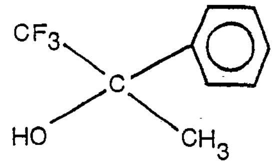

was obtained with a yield by 55% (1.25 gr). The boiling point of this carbinol was ascertained to be at 81 to 83°C/3 mmHg.

Example 4

Tetrahydrofuran (25 ml) was used as the solvent, and the solution was caused to react under the conditions identical to the Example 3, and methylphenyltrifluoromethylcarbinol was obtained with a yield by 43% (0.82 gr).

Examples 5 to 9

At the Example 1, for the carbonyl compounds to be used,
$C_5H_{11}CHO$ (Example 5),
$CH_3CH{=}CHCHO$ (Example 6),
$C_6H_5{-}CH{=}CHCHO$ (Example 7),

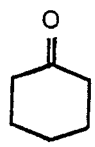 (Example 8),

$CH_2{=}CHCOCH_2CH_3$ (Example 9),

were substituted respectively, and the solution was caused to react under the condition identical to that of the Example 1, and varied types of carbinols as shown on the following Table 7 were obtained respectively. Further, the structure of each carbinol, including those of the Example 1 and 2, was identified by means of IR and NMR spectrography analysis.

TABLE 7

| Example | RCOR' | Reaction Time (hr) | Carbinol | | | |
|---|---|---|---|---|---|---|
| | | | Chemical Structure | Yield (%) | Boiling Point (°C/mmHg) | NHR $CF_3$ (δ) |
| 1 | $C_6H_5CHO$ | 0.5 | 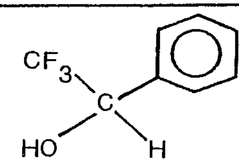 | 72 | 105 ~ 108/16 | -0.3* |
| 3 | $C_6H_5COCH_5$ | 1.0 | 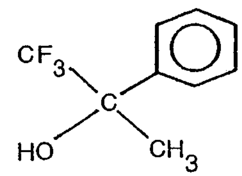 | 55 | 81 ~ 83/3 | -0.1 |
| 5 | $C_5H_{11}CHO$ | 0.5 | 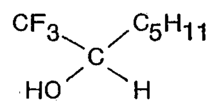 | 61 | 96 ~ 97/18 | 0.6 |
| 6 | $CH_3CH{=}CHCHO$ | 0.5 | 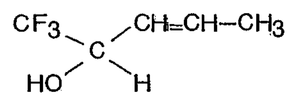 | 62 | 92 ~ 95/105 | 0.7 |
| 7 | $C_6H_5CH{=}CHCHO$ | 0.5 | 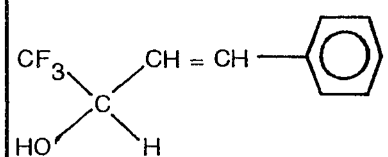 | 68 | 86 ~ 88/5 | 0.4 |
| 8 | 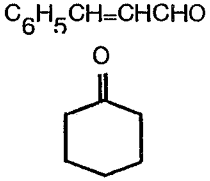 | 1.5 | 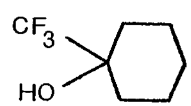 | 48 | 93 ~ 95/24 | -1.3 |
| 9 | 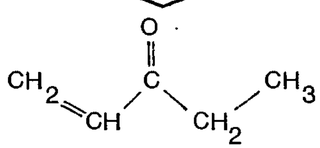 | 1.0 | 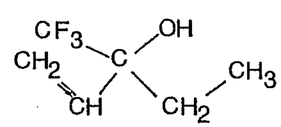 | 45 | 94 ~ 97/65 | 3.2 |

*$^{19}$F($CCl_4$):

δ α0.3 ($CF_3$ ($CF_3$, d, $JCF_3$—H = 5.6 Hz), $CF_3COOH$ to be the External Standard.

$^1$H($CCl_4$):

τ 5.00 (CH, q), 6.22 (OH), 7.50 (Ar—H).

Examples 10 to 17

At the Example 1, as the fluorine-containing aliphatic iodide and carbonyl compound, those indicated in the following Table 8 were used respectively, and caused the solution to react under the conditions identical to those of the Example 1. The amount used was; fluorine-containing aliphatic iodide: 11 mmol, carbonyl compound: 10 mmol, respectively.

For instance, where $(CF_3)_2CFI$ was used, the amount thereof was set at 3.26 gr (11 mmol) and phenyl (heptafluoro-1-methylethyl) carbinol was obtained by making the reaction product undergo the vacuum distillation, with a yield by 54% (1.49 gr). And the boiling point thereof was ascertained at 120 to 123°C/104 mmHg. For each example, the characteristics of the reaction substances and carbinol generated are summarized in the following Table 8.

TABLE 8

| Example | RCOR′ | $R_fI$ | Carbinol: Chemical Structure | Carbinol: Yield (%) | Carbinol: Boiling Point (°C/mmHg) |
|---|---|---|---|---|---|
| 10 | $C_6H_5CHO$ | $(CF_3)_2CFI$ | 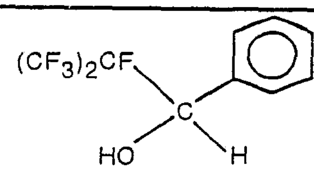 | 54 | 120 ~ 123/104 |
| 11 | $C_4H_9CHO$ | $(CF_3)_2CFI$ | 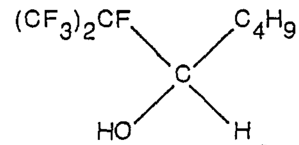 | 52 | 80 ~ 83/120 |
| 12 | $C_6H_5COCH_3$ | $(CF_3)_2CFI$ | 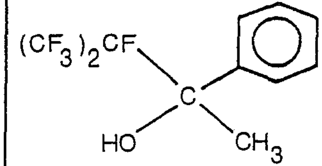 | 33 | 115 ~ 118/58 |
| 13 | $C_6H_5CHO$ | $CF_3(CF_2)_2I$ | 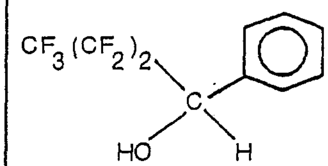 | 62 | 86 ~ 88/15 |
| 14 | $C_4H_9CHO$ | $CF_3(CF_2)_2I$ | 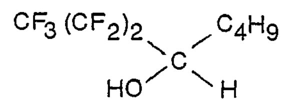 | 48 | 96 ~ 97/137 |
| 15 | $C_6H_5COCH_3$ | $CF_3(CF_2)_2I$ | 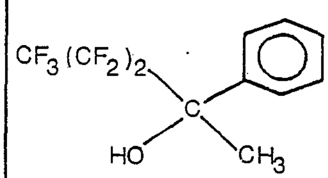 | 26 | 111 ~ 113/50 |
| 16 | $C_6H_5CHO$ | $CF_3(CF_2)_3I$ | 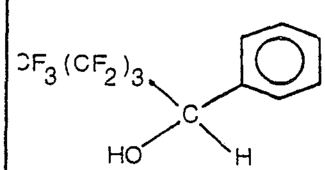 | 85 | 113/46 |
| 17 | $C_6H_5CHO$ | $CF_3(CF_2)_5I$ | 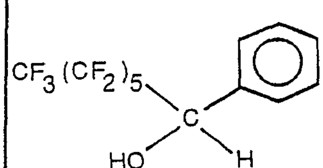 | 76 | 86 ~ 88/26 |

Example 18

At the Example 1, tin chloride ($SnCl_2$) was used instead of zinc powder, and caused the solution to react being acted with the ultrasonic waves, and the conditions identical to those of the Example 1, thereby the reaction product was hydrolyzed, then the similar phenyltrifluoromethylcarbinol was obtained.

Reference Example 19

At the Example 18, instead of giving the action of ultrasonic waves, pyridine (5 ml) was added, caused the solution to react under the conditions identical to those of the Example 18, thereby the reaction product was hydrolyzed, and the similar phenyltrifluoromethylcarbinol was obtained.

Comparative Example

For comparison, at the Example 1, magnesium powder was used instead of zinc powder, and the solution was caused to react under the conditions identical to those of the Example 1, it was ascertained that the desired fluorine-containing carbinol was not generated entirely. This similar result was confirmed where lithium powder was used.

Example 20

By putting trifluoromethyl and zinc powder into an autoclave respectively by the specified amount, and caused them to react under heating at 120 to 150°C, thereby $CF_3ZnI$ was obtained.

This substance was put into a flask together with benzaldehyde and dimethylformamide, as in the case of the Example 1, and subjected to the action of ultrasonic waves within a water bath. Upon completion of the reaction, 2% hydrochloric acid (100 ml) was added, and an oil layer thus obtained was extracted, and after drying, it was subjected to the vacuum distillation, thereby the phenyltrifluoromethylcarbinol was obtained similar to that of the Example 1.

Further, where $R_fZnI$ other than the above-given $CF_3ZnI$ was used, the correspoonding carbinols were obtained respectively.

Example 21

At the Example 20, by using tin chloride instead of zinc powder, it was caused to react with trifluoromethyl iodide ($SnCl_2$) as in the case of the Example 20, and $CF_3SnCl_2I$ was obtained. Consecutively, as in the case of the Example 20, $CF_3SnCl_2I$ was put into a flask together with benzaldehyde and dimethylformamide, and subjected to reaction under the action of ultrasonic waves, and the product extracted from an oil layer generated was hydrolyzed, thereby phenyltrifluoromethylcarbinol was obtained.

Reference Example 2

At the Example 21, instead of subjecting to the action of ultrasonic waves, under the condition where pyridine (5 ml) was being added, the object was caused to react, and the reacted product was hydrolyzed, thereby the similar phenyltrifluoromethylcarbinol was obtained.

Example 22

Commercially available zinc powder 1.3 gr (0.02 gr atom), trifluoromethyl inside 2.15 gr (11 mmol) cinnamylbromide 1.97 gr (10 mmol) and palladium acetate 0.11 gr (0.5 mmol) were put into a flask together with tetrahydrofuran 25 ml. Then, within a water bath, these substances were subjected to the reaction for a period of one hour under the action of ultrasonic waves by means of a commercially available ultrasonic cleaner (35W, 32 kHz). Thence, the solution within the flask was poured into the water, thereby an oil layer was further extracted by means of diethylether. After drying this extracted liquid with magnesium sulfate, the solvent was removed by means of fractional distillation.

By distillating residues, 3-trifluoromethyl-3-phenyl-1-propene ($ph(CF_3CHCH{=}CH_2)$ having a boiling point of 81 to 85°C/25 mmHG was obtained with a yield by 63%. The spectrum analysis value of this product was:

Mass: $M^+$ 186

IR: 1220 $cm^{-1}$ (C—F).

Example 23

At the Example 22, zinc powder 1.30 gr (0.02 gr atom), trifluoromethyl iodide 2.35 gr (12 mmol), trans-β-bromostyrene 1.83 g (10 mmol), and tetrakis (triphenylphosphine) palladium 0.23 gr (0.2 mmol) were used, and subjected to the reaction as in the case of the Example 22 within tetrahydrofuran (25 ml). When the product was treated as in the case of the Example 22, after distillation, trans-β-trifluoromethylstyrene ($PhCH{=}CHCF_3$) with the boiling point at 70 to 72°C/25 mmHg was obtained with a yield by 65%. The spectrum analysis values of this product were:

Mass: $M^+$ 172,

IR: 1215 $cm^{-1}$ (C—F).

Example 24

At the Example 22, zinc powder 1.30 gr (0.02 gr atom), benzene iodide 2.04 gr (10 mmol), heptafluoro-1-methyl ethyl iodide 2.26 gr (11 mmol), and bis(triphenylphosphine) palladium dichloride 0.07 gr (0.1 mmol) were used, and subjected to the reaction for 30 minutes within tetrahydrofuran (25 ml) under the action of ultrasonic waves. Then, by treating the product similarly to the Example 22, and by means of distillation, heptafluoro-1-methylethyl benzene

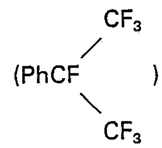

with the boiling point at 124 to 126°C was obtained with a yield by 87%. The spectrum analysis values were:

Mass: $M^+$ 246

IR: 1130 to 1310 $cm^{-1}$ (C—F).

Example 25

At the Example 22, zinc powder 1.30 gr (0.02 gr atom), trifluoromethyl iodide 2.35 gr (12 mmol), 1-tolyl-3-bromo-1-propene 2.11 gr (10 mmol), and palladium acetate 0.11 gr (0.5 mmol) were subjected to the reaction similar to that of the Example 22 within tetrahydrofuran 25 ml.

The product was treated as in the case of the Example 22, after distillation, 3-trifluoromethyl-3-tolyl-1-propene ($4—CH_3C_6H_4(CF_3)CHCH_3 = CH_2$) with a boiling point at 90 to 92°C/26 mmHG was obtained with a yield by 67%. The spectrum analysis values were:

Mass: $M^+$ 200,

IR: 1215 $cm^{-1}$ (C—F).

Example 26

At the Example 22, zinc powder 1.30 gr (0.02 gr atom), trifluoromethyl iodide 2.15 gr (11 mmol), 1-bromo-2-tolyl ethene 1.97 gr (10 mmol), and tetrakis (triphenylphosphine) palladium 0.23 gr (0.2 mmol) were used to be subjected to the reaction within tetrahydrofuran (25 l) as in the case of the Example 22. The product was treated similarly to the Example 22, and was recrystallized by means of petroleum ether, thereby 1-trifluoromethyl-2-tolyl-ethene $4—CH_3C_6H_4CH{=}CHCF_3$ with a melting point at 59 to 60°C was obtained with a yield by 67%. The spectrum analysis values of this product were:

Mass: $M^+$ 186

IR: 1210 $cm^{-1}$ (C—F).

Example 27

At the Example 22, zinc powder 1.30 gr (0.02 gr atom), heptafluoro-1-methylethyl iodide 2.26 gr (11 mmol) cinnamylbromide 1.97 gr (10 mmol), and palladium acetate 0.11 gr (0.5 mmol) were subjected to the reaction within tetrahydrofuran (25 ml) as in the case of the Example 22. When the product was treated similarly to the Example 22, 3-phenyl-3-(heptafluoro-1-methylethyl)-1-propene ($Ph(C_3F_7)CHCH{=}CH_2$) with a boiling point at 80 to 82°C/21 mmHG was obtained with a yield by 78%. The spectrum analysis values were:

Mass: $M^+$ 286,

IR: 1120 to 1310 $cm^{-1}$ (C—F).

Example 28

By putting the specified amount of trifluoromethyl iodide and zinc powder respectively into an autoclave, they were subjected to the reaction being heated at 120 to 150°C, and trifluoromethyl zinc iodide ($CF_3ZnI$) was obtained. This $CF_3ZnI$ was put into a flask together with cinnamylbromide palladium diacetate and tetrahydrofuran, as in the case of the Example 22, and was subjected to the reaction under the action of ultrasonic waves.

When the solution thus obtained was treated similarly to the Example 22, after distillation, 3-trifluoromethyl-3-phenyl-1-propene similar to that of the Example 22 was obtained.

Example 29

1) Synthesis of $CF_3COCH_2COCF_3$

By putting $CF_3COCH_2Br$ (1.91 gr, 10 mmol), $CF_3CO_2C_2H_5$ (1.42 gr, 10 mmol), and zinc powder (1.3 gr) into a flask (100 ml) together with a mixed solvent comprising tetrahydrofuran (20 ml) and dimethylformamide (10 ml), they were subjected to the reaction for a period of one hour being irradiated with ultrasonic waves by means of an ultrasonic cleaner (35W, 32 kHz). After the reaction, impurities contained in the mixed liquid were filtered, then the water (500 ml) was added to the filtered liquid, thereby an oil layer generated was extracted by means of diethylether. After drying the extracted liquid with magnesium sulfate, the solvent was removed. The product ($CF_3COCH_2COCF_3$) (bp: 70 to 72°C) was obtained through distillation with a yield by 69%.

2) Synthesis of $CF_3COCH_2COCF(CF_3)OCF_2CF_2CF_3$

$CF_3COCH_2Br$ (1.91 gr, 10 mmol), $CF_3CF_2CF_2OCF(CF_3)CO_2C_2H_5$ (3.58 gr, 10 mmol), and zinc powder (1.3 gr) were put into a flask (100 ml) together with a mixed solvent comprising tetrahydrofuran (20 ml) and dimethylformamide (10 ml), and they were subjected to the reaction for a period of one hour under the action of ultrasonic waves by means of an ultrasonic cleaner (35W, 32 kHz).

After the reaction, impurities within a mixed solution were filtered, and the filtered liquid was added with the water (500 ml), and an oil layer thus generated was extracted with diethylether. After drying the extracted liquid with magnesium sulfate, the solvent was removed through fractional distillation. The product (bp: 80 to 83°C/150 mmHg) was obtained by means of distillation with a yield by 58%. The spectrum analysis values were:

Mass:
$M^+$ 440
NMR:
$^1H$ (δ) ($CDCl_3$),
5.2 ($CH_2$) ppm,
$^{19}F$ (δ),
−11 ($CF_3CO$),
1.6 (1F),
4.2 (3F),
4.8 (3F),
6.6 (1F),
52 (2F),
58 (1F) ppm
(External Standard: $CF_3CO_2H$)

Example 30

1) Synthesis of $C_2H_5OCOCH_2COCF_3$

$BrCH_2CO_2CH_2CH_3$ (1.67 gr, 10 mmol), $CF_3CO_2CH_2CH_3$ (1.42 gr, 10 mmol), and zinc powder (1.3 gr) were put into a flask (100 ml) together with a mixed solvent comprising tetrahydrofuran (20 ml) and dimethylformamide (10 ml), and they were subjected to the reaction for a period of one hour under the irradiation of ultrasonic waves by means of an ultrasonic cleaner (35W, 32 kHz). After the reaction, impurities contained in the mixed liquid were filtered, and the water (500 ml) was added to the filtered liquid. And an oil layer thus generated was extracted with diethylether. After drying the extracted liquid with magnesium sulfate, the solvent was removed through fractional distillation. Thereby the product (bp: 70 to 72°C/102 mmHg) was obtained by means of the vacuum distillation with a yield by 83%.

2) Synthesis of $C_2H_5OCOCHFCOCF_3$

Ethyl bromofluoroacetate ($BrCHFCO_2C_2H_5$; 1.85 gr, 10 mmol), $CF_3COOC_2H_5$ (1.42 gr, 10 mmol), and zinc powder (1.3 gr) were put into a flask (100 ml) together with a mixed solvent comprising tetrahydrofuran (20 ml) and dimethylformamide (10 ml), and subjected to the reaction for a period of one hour being irradiated with ultrasonic waves by means of an ultrasonic cleaner (35W, 32 kHz). After the reaction, impurities contained in the mixed liquid were filtered, and the water was added to the filtered liquid, and an oil layer thus generated was extracted with diethylether. After drying the extracted liquid by means of magnesium sulfate, the solvent was removed through fractional distillation. Then the product (bp: 72 to 74°C/96 mmHg) was obtained by means of the vacuum distillation. The spectrum analysis values were:

Mass:
$M^+$ 202
NMR:
$^1H$ (δ) ($CDCl_3$),
1.0 ($C\underline{H}_3CH_2$),
2.3 ($CH_3C\underline{H}_2$),
4.6 ($C\underline{H}$) ppm
$^{19}F$ (δ),
−13 ($CF_3$),
+116 (CF) ppm
(External Standard: $CF_3CO_2H$)

Example 31

1) Synthesis of $C_2H_5OCOCH_2COC_2F_5$

$BrCH_2CO_2C_2H_5$ (1.67 gr, 10 mmol), $C_2F_5CO_2C_2H_5$ (1.92 gr, 10 mmol), and zinc powder (1.3 gr) were put into a flask (100 ml) together with a mixed solvent comprising tetrahydrofuran (20 ml) and dimethylformamide (10 ml) and treated similarly to the Example 30, and the product (bp: 83 to 85°C/91 mmHg) was obtained by means of the vacuum distillation with a yield by 86%. The spectrum analysis values were:

Mass:
$M^+$ 234
NMR: $^1H$ (δ) ($CDCl_3$)
1.1 ($C\underline{H}_3CH_2$),
2.2 ($CH_3CH_2$),
4.1 ($CH_2$) ppm
$^{19}F$ (δ)

1.6 ($CF_3$)
29 ($CF_2$) ppm
(External Standard: $CF_3CO_2H$)

2) Synthesis of $C_2H_5OCOCHFCOC_2F_5$

At the above Experiment 1, by using $B_rCHFCOOCH_2CH_3$ (1.85 gr, 10 mmol), $C_2F_5CO_2C_2H_5$ (1.92 gr, 10 mmol), and zinc powder (1.3 gr), and tetrahydrofuran (20 ml) and dimethylformamide (10 ml), as the solvents, the treatment similar to that of Para 1) was conducted. Through the vacuum distillation, the product (bp: 78 ~ 80°C/80 mmHg) was obtained with a yield by 74%. The spectrum analysis values were:

Mass:
$M^+$ 252
NMR:
$^1H$ (δ) ($CDCl_3$),
1.1 ($CH_3CH_2$)
2.1 ($CH_3CH_2$),
4.5 (CH) ppm
$^{19}F$ (δ)
1.4 ($CF_3$),
32 ($CF_2$),
114 (CF) ppm
(External Standard: $CF_3CO_2H$)

Example 32
Synthesis of

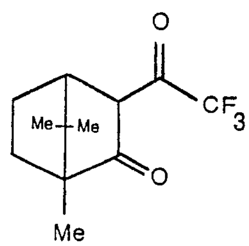

1) d-3-bromcamphor (4,62 gr, 10 mmol), $CF_3COOC_2H_5$ (3.1 gr, 22 mmol), and magnesium (0.53 gr) were mixed with diethylether (30 ml), and put into a flask (100 ml), and they were subjected to the reaction for a period of 40 minutes being irradiated with ultrasonic waves by means of an ultrasonic cleaner (35W, 32 kHz). After the reaction impurities contained in the mixed liquid were filtered, and 2% HCl water solution (500 ml) was added, and an oil layer thus generated was extracted with diethylether. After cleaning the extracted liquid with saturated water solution ($NaHCO_3$), it was dried with magnesium sulfate. After removal of the solvent by means of fractional distillation, the product (bp: 76 to 80°C/5 mmHg) was obtained through the vacuum distillation with a yield by 94%. The spectrum analysis values of this product were:

NMR:
$^1H$ (δ) ($CCl_4$),
0.85 (3H)
1.0 (6H),
1.2 ~ 2.3 (5H),
2.85 (1H) ppm
$^{19}F$ (δ)
−24 ($CF_3$) ppm
(External Standard: $CF_3CO_2H$).

2) At the reaction of the above para 1), d-3-bromcamphor (4.62 gr, 20 mmol), $CF_3COOC_2H_5$ (3.1 gr, 22 mmol), and zinc powder (2.6 gr) were mixed with tetrahydrofuran (30 ml), and put into a flask (100 ml), then they were subjected to the reaction for a period of one hour under the action of ultrasonic waves by means of an ultrasonic cleaner (35W, 32 kHz). After the reaction, impurities contained in a mixed liquid was filtered, and the water (500 ml) was added to the filtered liquid, and an oil layer was extracted with diethylether. After drying with magnesium sulfate, the solvent was removed by means of fractional distillation, thence the product was obtained through the vacuum distillation with a yield by 86%.

Reference Example 3

1) Synthesis of optically active perfluoro-2-propoxypropionic acid

Perfluoro-2-propoxypropionic acid fluoride (13.28 gr, 40 mmol) was dropped, within the water bath, into a dichloromethane solution (50 ml) containing (−)-α-phenylethylamine (48.4 gr, 40 mmol) and triethylamine (4.04 gr, 40 mmol). After dropping, the mixture was further stirred up for a period of 30 minutes under the room temperature. The mixture reacted was cleaned with the water and 1 normal hydrochloric acid, and after drying, the solvent was removed, thereby the oily diastereomer mixture ((+)—(−) and (−)—(−)) was obtained.

This mixture was separated by means of the mixed solvent comprising hexane and benzene (3:1) within a silica gel column. (−)—(−) amide (6.23 gr, 72%) was obtained as the first fraction. This amide was ascertained to have a melting point, 55.5 to 56.5°C, $[\alpha]_D^{20}$ −82.0° (c1.00, $C_6H_6$). And as the second fraction, 6.06 gr (70%) of (+)—(−) amide with a melting point, 83 to 83.5°C, $[\alpha]_D^{20}$ −88.0° (c 1.00, $C_6H_6$) was obtained.

Both of them, at the IR spectrum, presented the characteristic absorption of NH and C=O at 3300 $cm^{-1}$ and 1700 $cm^{-1}$ respectively. Also at $^1H$ NMR (in $CCl_4$), both of them presented the entirely identical patterns; (δ 1.53 (Me), 5.12 (CH), 6.72 (NH), 7.27 (Ph)).

However, for $^{19}F$ NMR, at the chemical shift of the terminal trifluoromethyl group of perfluoropropoxy group, it was found out that (−)—(−) amide was presented at the lower magnetic field of 9 Hz compared with (+)—(−) amide.

Also, at the gas chromatography, both of them were different in their retention time, and the purity could be determined by means of $^{19}F$ NMR and gas chromatography.

The pure (+)—(−) amide 4.33 gr (10 mmol) was added into the solvent (40 ml) which was a mixture of water and ethanol containing 10% of sodium hydroxide, and subjected to heating and reflux for a period of 16 hours. The reaction liquid was neutralized with hdyrochloric acid, and carboxylic acid thus generated was extracted by means of ether. After drying the extracted liquid with magnesium sulfate, ether was removed, and by subjecting the residue to the vacuum distillation, (+)-perfluoro-2-propoxypropionic acid (2.28 gr, 69%) was obtained. ($\alpha_D^{28}$ +26.50° (neat, l=1)).

Similarly, from (−)—(−) amide, (−)-perfluoro-2-propoxypropionic acid (2.38 gr, 72%) was obtained. ($\alpha_D^{28}$ −26.30° (neat, l=1)).

The material-property values, IR, and NMR spectrum values of both of them are given as follows:

Boiling point:
93 ~ 94°C/90 mmHg,
IR (film):
3200 (OH), 1780 (C=O) $cm^{-1}$
$^1H$ NMR ($CDCl_3$):
δ 10.3 ppm
$^{19}F$ NMR (neat):
δ 2.7 (1F),
5.43 (3F),
6.3 (3F),
10.0 (1F),
53.0 (2F),
54.8 1F) ppm
(External Standard: $CF_3CO_2H$)

2) Synthesis of (+)-perfluoro-2-propoxypropionic acid chloride

(+)-perfluoro-2-propionic acid 3.30 gr (10 mmol) was dropped into phosphorus pentachloride 2.50 gr (12 mmol) in ice bath. Upon ocmpletion of that addition, the susbtance was stirred up for 10 minutes under the room temperature, and through careful distillation, acid chloride 3.24 gr (93%) was obtained. The boiling point; 73 to 74°C, $\alpha_D^{20}$ +8.66° (neat, l=1).

3) Synthesis of (+)-di(perfluoro-2-propoxypropionyl) methane

Isopropyl magnesium chloride (40 mmol) was synthesized in diethylether (20 ml). To the substance, acetate tert-butyl 4.64 gr (40 mmol) was dropped under the room temperature. The reaction was exothermic and propane gas was rapidly generated. Dropping rate was adjusted so as to make the ether self-refluxed. Upon completion of dropping, after the substance was further stirred up for a period of one hour, (+)-perfluoro-2-propoxypropionic acid chloride 6.54 gr (18 mmol) was dropped.

After the dropping, the substance was stirred up for a period of 15 minutes under the room temperature, thereafter it was quenched with dilute hydrochloric acid. After separating an organic layer, the substance was dried with magnesium sulfate. After removing the ether and excessive reagent by means of fractional distillation, a small amount of p-toluenesulfonic acid was added, and the mixture was heated at 120°C for a period of 15 minutes. The material-property values and spectrum analysis values were:

Yield:
5.20 gr (90%)

Boiling point:
$94 \sim 96°C/110$ mmHg
$[\alpha]_D^{28}$:
+34.88° (neat, l=1)
IR (film):
1600 $cm^{-1}$ (C=O)
$^1H$ NMR ($CCl_4$):
13.67, 5.97 ppm
$^{19}F$ NMR ($CCl_4$):
δ 1.65 (1F),
4.00 (3F),
4.77 (3F),
6.55 (1F),
51.73 (2F),
57.50 (1F) ppm
(External Standard: $CF_3CO_2H$)
Cu chelate:
m.p.: $71 \sim 72°C$

Reference Example 4

Europium chloride·6 hydrate 0.73 gr (2 mmol) was dissolved into ethanol (7 ml). Into 50% water solution (6 ml) of 1 normal sodium hydroxide which was added with 3.84 gr (6 mmol) of (+)-di(perfluoro-2-propoxypropionyl) methane, europium solution was added, and the mixture was stirred up for a period of 2 hours under the room temperature. After that treatment, the distilled water (10 ml) was added, and oil substance thus separated was extracted by means of pentane. After washing the pentane-extracted liquid with water, it was dried with magnesium sulfate.

When the solvent was removed through the vacuum distillation, and leaving the substance as it was for a period of several hours under a temperature at 90°C (0.8 mmHg), a yellow oily substance was obtained.

It was ascertained that it was an almost pure substance as desired (europium chelate) when it was examined with IR and NMR spectrum, the values below were measured:
Yield:
3.22 gr (78%)
IR (film):
1639 (C=O), 1530 (C=C) $cm^{-1}$
$^1H$ NMR ($CF_2ClCFCl_2$):
δ 3.33 ppm
$^{19}F$ NMR ($CF_2ClCFCl_2$):
δ 6.90 (6F),
7.57 (2F),
54.47 (2F),
61.83 (1F) ppm
External Standard: $CF_3CO_2H$)
$[\alpha]_D^{26}$: +48.52° (C 0.67, $CF_2ClCFCl_2$)

Example 33

Synthesis of $CF_3COCH_2COCF(CF_3)OCF_2CF_2CF_3$

$CF_3COCH_2Br$ (1.91 gr, 10 mmol), $CF_3CF_2CF_2OCF(CF_3)CO_2C_2H_5$ (3.58 gr, 10 mmol), and zinc powder (1.3 gr) were put into a flask (100 ml) together with a mixture comprising tetrahydrofuran (20 ml) and diethylformamide (10 ml), and subjected to the reaction for a period of one hour under the irradiation of ultrasonic waves generated by an ultrasonic cleaner (35W, 32 kHz).

After the reaction, impurities contained in the mixed liquid were filtered, and the water (500 ml) was added to the filtered liquid, and an oily layer thus generated was extracted. After drying the extracted liquid with magnesium sulfate, the solvent was removed by means of fractional distillation. Through the distillation, the product (bp: 80 to 83°C/150 mmHg) was obtained with a yield by 58%. The spectrum analysis values of this product were:
Mass:
$M^+$ 440
NMR:
$^1H$ (δ $CDCl_3$)
5.2 ($CH_2$) ppm
$^{19}F$ (δ)
−11 ($CF_3CO$),
1.6 (1F),
4.2 (3F),

4.8 (3F),
6.6 (1F),
52 (2F),
58 (1F) ppm
(External Standard: $CF_3CO_2H$)

## Claims

1. A process for producing fluorine-containing organic compounds characterized in that fluorine-containing aliphatic iodides which comprise at least one selected from the group of $R_fI$, $R_fZ_nI$ and $R_fS_nX_2I$ (where $R_f$ indicates a fluorine-containing aliphatic group; and X indicates halogen), or which comprise $R_fCOOR'$ (where $R_f$ indicates a fluorine-containing aliphatic group; and R' indicates an aliphatic hydrocarbon group or aromatic hydrocarbon group) are reacted with organic compounds under ultrasonic wave action, thereby the $R_f$ group or the $R_fCO$ group is introduced into the said organic compounds.

2. A process according to claim 1; wherein a carbonyl compound which is represented by the Formula: RCOR' (where R and R' represent similar or dissimilar atoms or groups which are selected from the groups comprising the hydrogen atoms, aliphatic groups, and aromatic groups, and where both of them are of the same aliphatic group, they are capable to form rings jointly) is used as said organic compound, and is caused to react with said fluorine-containing aliphatic iodide under the presence at least of either zinc powder and halogenated tin powder which is represented by $SnX_2$ (where X represents halogens).

3. A process according to claim 1; wherein said fluorine-containing aliphatic iodide is caused to react with a halogen-substituted unsaturated compound having multiple bonds between carbon atoms, or a halogen-substituted aromatic compound under the action of ultrasonic waves under the presence of zinc powder and palladium-type catalyst, thereby said $R_f$ is introduced into said multiple bond or benzene nucleus, and thereby an aliphatic unsaturated compound or an aromatic compound which is eliminated of said halogen is obtained.

4. A process according to claim 3; wherein, as a halogen-substituted aliphatic unsaturated compound, a halogenated allyl compound or its derivative which is represented by the Formula:

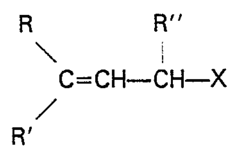

(where R, R' and R'' represent an hydrogen atom, an alkyl group or alkenyl group, having carbon atom numbers of 10 or less than 10, aromatic group or aromatic group substituted with a substituent and X represents halogen.), or a halogenated vinyl compound or its derivative which is represented by the Formula:

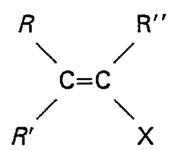

(where R, R' and R'' are the same as those described above, and X represents halogen) is used; and as a halogen-substituted aromatic compound, a halogenated aryl compound or its derivative which is represented by the Formula:

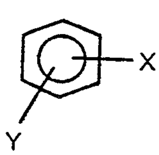

(where X represents halogens; and Y represents at least one type of a hydrogen atom or substituents which are selected from groups comprising an alkyl group or alkenyl group having carbon atom numbers of 5 or less than 5, or cyano group, nitro group, ester group and aryl group) is used.

5. A process according to claim 3 or claim 4; wherein a divalent or 0-valent palladium compound is used as a palladium-type catalyst.

6. A process according to any of claim 1 through 5; wherein $R_f$ is a fluorine-containing alkyl group or alkenyl group having carbon atoms in the number of 10 or less.

7. A process according to claim 2 or claim 6; wherein R and R′ are an alkyl group or alkenyl group having carbon atoms in the number of 15 or less.

8. A process according to any of claim 1 through claim 7; wherein zinc powder and/or halogenated tin powder are used within a range of one to three times $R_fI$, in the number of moles.

9. A process according to any of claim 1 through claim 8; wherein fluorine-containing aliphatic iodide and a carbonyl compound or a halogen-substituted aliphatic or aromatic compound are caused to react under the use of aprotic polar solvent.

10. A process according to any claim 1 through claim 9; wherein the reaction is caused under the ambient temperature and atmospheric pressure.

11. A process according to claim 1, wherein fluorine-containing aliphatic iodide which is represented by the Formula:

$$R_fZ_nI \text{ or } R_fS_nX_2I$$

(where $R_f$ represents fluorine-containing aliphatic groups; and X represents halogens.) is caused to react with a carbonyl compound which is represented by the formula:

$$RCOR'$$

(where R and R′ represent similar or dissimilar atoms or groups which are selected from groups comprising hydrogen atoms, aliphatic groups or aromatic groups, and where both of them are of aliphatic groups, they are capable to form rings jointly.).

12. A process according to claim 11; wherein said fluorine-containing aliphatic zinc iodide is subjected to the reaction with a halogen-substituted aliphatic unsaturated compound having multiple bonds between carbon atoms, or a halogen-substituted aromatic compound under the action of ultrasonic waves and under the presence of a palladium-type catalyst, thereby an aliphatic unsaturated compound or aromatic compound which is introduced with said $R_f$ into said multiple bonds or benzene nucleus, and eliminated of said halogen, is obtained.

13. A process according to claim 12, wherein as a halogen-substituted aliphatic unsaturated compound, a halogenated allyl compound or its derivative which is represented by the Formula:

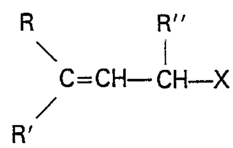

(where R, R′ and R″ represent a hydrogen atom, an alkyl group or alkenyl group, having carbon atom numbers of 10 or less than 10, or aromatic group or aromatic group which is substituted with a substituent; and X represents halogens.), or a halogenated vinyl compound or its derivative which is represented by the Formula:

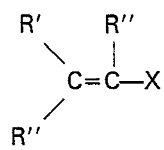

(where R, R′ and R″ are the same as those described before; and X represents halogens.) is used, and as a halogen-substituted aromatic compound, a halogenated aryl compound or its derivative which is represented by the Formula:

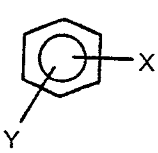

(where X represents halogens; and Y represents a hydrogen atom or at least one of substituents which is selected from groups comprising an alkyl group or alkenyl group, having carbon atom numbers of 5 or less than 5, cyano group, nitro group, ester group and aryl group is used.

14. A process according to any of claim 12 or claim 13; wherein, as palladium-type catalyst, a divalent or O-valent palladium compound is used.

15. A process according to any of claim 11 through claim 14; wherein $R_f$ represents a fluorine-containing alkyl group or fluorine-containing alkenyl group having carbon atoms in the number of 10 or less.

16. A process according to claim 11 or claim 15; wherein R and R′ are to be an alkyl group or alkenyl group having carbon atoms in the number of 15 or less.

17. A process according to any of claim 11 through 16; wherein fluorine-containing aliphatic iodide is caused to react with a carbon compound or halogen-substituted aliphatic or aromatic compound under the use of aprotic polar solvent.

18. A process according to any of claim 11 through claim 17; wherein the reaction is caused under the ambient temperature and atmospheric pressure.

19. A process according to any of claim 11 through 18; wherein fluorine-containing aliphatic iodide which is represented by the formula:

$$R_fI$$

(where $R_f$ represents a fluorine-containing aliphatic group) is caused to react with zinc powder and/or halogenated tin powder which is represented by $SnX_2$ (where X presents halogens) under heating, thereby $R_fZnI$ and/or $R_fSnX_2I$ are (is) generated, thence the product(s) is used for the reaction.

20. A process according to claim 19; wherein zinc powder is used within a range of one to three times that of $R_fI$ in the number of moles.

21. A process according to claim 1, having a process;

1) wherein halogenated ketone which is represented by the Formula:

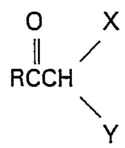

(where R represents an aliphatic hydrocarbon-oxy group, aliphatic hydrocarbon group or fluoro aliphatic hydrocarbon group, and includes those which are forming rings between carbon atoms being bonded with X; X represents halogen; and Y represents hydrogen atoms or fluorine atoms.) is caused to react with a metal (M) comprising zinc or magnesium, thereby generates a metallic halogenated ketone which is represented by the Formula:

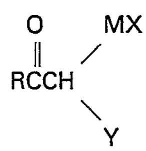

(where R, X and Y are the same as those described before; and M represents zinc or magnesium); and process;

2) wherein the metallic halogenated ketone thus generated is caused to react with a fluoroester which is represented by the Formula:

$$R_fCOOR'$$

(where $R_f$ represents a fluoroaliphatic hydrocarbon group, and includes those having ether bonds in their chains; R′ represents an aliphatic hydrocarbon group or aromatic hydrocarbon group) under the action of ultrasonic waves, thereby generates a fluorine-containing diketone which is represented by the Formula:

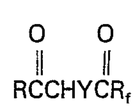

(where R, Y and $R_f$ are the same as those described before.)

22. A process according to claim 21; wherein a halogenated ketone is caused to react with a fluoroalkylester under the action of ultrasonic waves under the presence of zinc or magnesium.

23. A process according to claim 21; wherein a halogenated ketone is caused to react with zinc or magnesium under the action of ultrasonic waves to generate metallic halogenated ketone, and further this metallic halogenated ketone is caused to react with fluoroalkylester under the action of ultrasonic waves.

24. A process according to claim 21; wherein metallic halogenated ketone is generated by making halogenated ketone and zinc or magnesium to react under heating, and further this metallic halogenated ketone is subjected to reaction with fluoroalkyl ester under the action of ultrasonic waves.

25. A process according to any of claim 21 through claim 24; wherein, in at least one of aprotic polar solvents which is selected from a group comprising tetrahydrofuran, dimethylformamide, acetonitrile, dimethylsulfoxide, dimethylacetamide, N-methylpyrrolidone and hexamethylphosphoamide, the reaction is caused to take place.

26. A process according to any of claim 21 through claim 25; wherein the number of carbon atoms of R is limited to 10 or less.

27. A process according to claim 26; wherein, as halogenated ketone, d-3-halogenated camphor is used.

28. A process according to any of claim 21 through claim 27; wherein X is to be of bromine atoms or iodine atoms.

29. A process according to any of claim 21 through claim 28; wherein $R_f$ is to be of a fluoroalkyl group or alkenyl group, or fluoroalkyl group or alkenyl group which is substituted and introduced with other halogens, all of which having carbon atoms in the number of 15 or less.

30. A process according to any of claim 21 through claim 28; wherein $R_f$ is to be of a fluoroalkylether group which is represented by the Formula:

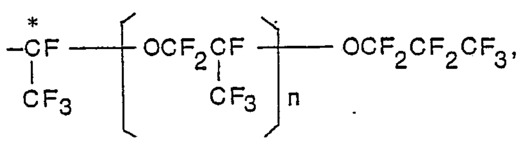

(where n = 0 ~ 3 ; and $\overset{*}{C}$ represents asymmetric carbon.)

31. A process according to any of claim 21 through claim 30; wherein R′ is to be an alkyl group or alkenyl group having carbon atoms in the number of 10 or less.

**Patentansprüche**

1. Verfahren zur Herstellung von fluorhaltigen organischen Verbindungen, dadurch gekennzeichnet, daß fluorhaltige, aliphatische Iodide, die mindestens einen Vertreter der Gruppe $R_fI$, $R_fZnI$ und $R_fSnX_2I$ (worin $R_f$ für eine fluorhaltige aliphatische Gruppe und X für Halogen stehen) oder der Formel $R_fCOOR'$ (worin $R_f$ für eine fluorhaltige aliphatische Gruppe und R′ für eine aliphatische Kohlenwasserstoffgruppe oder eine aromatische Kohlenwasserstoffgruppe stehen) umfassen, unter Einwirkung von Ultraschallwellen mit organischen Verbindungen umgesetzt werden, um die $R_f$-Gruppe oder die $R_fCO$-Gruppe in die organischen Verbindungen einzuführen.

2. Verfahren nach Anspruch 1, dadurch gekennzeichnet, daß eine Carbonylverbindung der Formel: RCOR′ (worin R und R′ gleichartige oder verschiedene Atome oder Gruppen ausgewählt aus Wasserstoffatomen, aliphatischen Gruppen und aromatischen Gruppen darstellen, wobei dann, wenn beide Gruppen aliphatische Gruppen darstellen, diese gemeinsam Ringe bilden können) als organische Verbindung verwendet wird und in Gegenwart von mindestens einem Vertreter der Zinkpulver und halogeniertes Zinnpulver, welches durch die Formel $SnX_2$ (worin X Halogene darstellt) wiedergegeben werden kann, mit dem fluorhaltigen aliphatischen Iodid umgesetzt wird.

3. Verfahren nach Anspruch 1, dadurch gekennzeichnet, daß das fluorhaltige aliphatische Iodid mit einer halogensubstituierten ungesättigten Verbindung, die Mehrfachbindungen zwischen Kohlenstoffatomen aufweist, oder einer halogensubstituierten aromatischen Verbindung in Gegenwart von Zinkpulver und eines Katalysators des Palladium-Typs unter Einwirkung von Ultraschallwellen umgesetzt wird, um die $R_f$-Gruppe in die Mehrfachbindung oder den Benzolkern einzuführen und dabei eine aliphatische ungesättigte Verbindung oder eine aromatische Verbindung, von der das Halogen abgespalten ist, zu bilden.

4. Verfahren nach Anspruch 3, dadurch gekennzeichnet, daß als halogensubstituierte, aliphatische, ungesättigte Verbindung eine halogenierte Allylverbindung oder ein Derivat davon der Formel

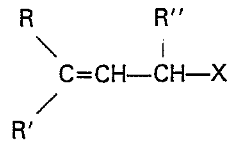

(worin R, R' und R'' Wasserstoffatome, Alkylgruppen oder Alkenylgruppen mit 10 oder weniger als 10 Kohlenstoffatomen, aromatische Gruppen oder mit einem Substituenten substituierte aromatische Gruppen und X Halogen bedeuten) oder eine halogenierte Vinylverbindung oder ein Derivat davon der Formel

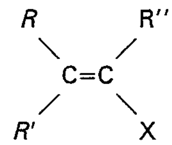

(worin R, R' und R'' die oben angegebenen Bedeutungen besitzen und X halogen darstsellt) verwendet wird und als halogensubstituierte aromatische Verbindung eine halogenierte Arylverbindung oder ein Derivat davon der Formel

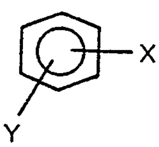

(worin X Halogene und Y mindestens einen Vertreter der Wasserstoffatome oder Substituenten aus der Alkylgruppen oder Alkenylgruppen mit 5 oder weniger als 5 Kohlenstoffatomen, Cyanogruppen, Nitrogruppen, Estergruppen und Arylgruppen umfassenden Gruppe darstellen) verwendet wird.

5. Verfahren nach Anspruch 3 oder 4, dadurch gekennzeichnet, daß als Katalysator des Palladium-Typs eine zweiwertige oder nullwertige Palladiumverbindung eingesetzt wird.

6. Verfahren nach einem der Ansprüche 1 bis 5, dadurch gekennzeichnet, daß $R_f$ für eine fluor-haltige Alkylgruppe oder Alkenylgruppe mit 10 oder weniger Kohlenstoffatomen steht.

7. Verfahren nach den Ansprüchen 2 oder 6, dadurch gekennzeichnet, daß R und R' alkylgruppen oder Alkenylgruppen mit 15 oder weniger Kohlenstoffatomen stehen.

8. Verfahren nach einem der Ansprüche 1 bis 7, dadurch gekennzeichnet, daß Zinkpulver und/oder halogeniertes Zinnpulver in einer Menge von 1 bis 3 Mol pro Mol $R_fI$ eingesetzt werden.

9. Verfahren nach einem der Ansprüche 1 bis 8, dadurch gekennzeichnet, daß ein fluorhaltiges, aliphatisches Iodid und eine Carbonylverbindung oder eine halogensubstituierte, aliphatische oder aromatische Verbindung unter Anwendung eines aprotischen polaren Lösungsmittels umgesetzt werden.

10. Verfahren nach einem der Ansprüche 1 bis 9, dadurch gekennzeichnet, daß die Reaktion bei Umgebungstemperatur und Atmosphärendruck durchgeführt wird.

11. Verfahren nach Anspruch 1, dadurch gekennzeichnet, daß ein fluorhaltiges, aliphatisches Iodid der Formel

$$R_fZnI \text{ oder } R_fSnX_2I$$

(worin $R_f$ fluorhaltige aliphatische Gruppen und X Halogene darstellen) mit einer Carbonylverbindung der Formel

$$RCOR'$$

(worin R und R' gleichartige oder verschiedene Atome oder Gruppen aus der Wasserstoffatome, aliphatische Gruppen oder aromatische Gruppen umfassenden Gruppe, wobei dann, wenn beide Gruppen aliphatische Gruppen darstellen, diese gemeinsam Ringe bilden können) umgesetzt wird.

12. Verfahren nach Anspruch 11, dadurch gekennzeichnet, daß das fluorhaltige, aliphatische Zinkiodid mit einer halogensubstituierten, aliphatischen ungesättigten Verbindung mit Mehrfachbindungen zwischen Kohlenstoffatomen, oder einer halogensubstituierten aromatischen Verbindung in Gegenwart von Ultraschallwellen und eines Katalysators des Palladium-Typs umgesetzt wird, um eine aliphatische ungesättigte Verbindung oder eine aromatische Verbindung, in deren Mehrfachbindung oder deren benzolkern die Gruppe $R_f$ eingeführt und das Halogen abgespaltet ist, zu erhalten.

13. Verfahren nach Anspruch 12, dadurch gekennzeichnet, daß daß als halogensubstituierte, aliphatische, ungesättigte Verbindung eine halogenierte Allylverbindung oder eine Derivat davon der Formel

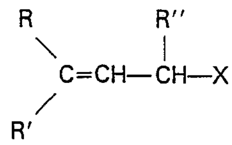

(worin R, R′ und R′′ Wasserstoffatome, Alkylgruppen oder Alkenylgruppen mit 10 oder weniger als 10 Kohlenstoffatomen, aromatische Gruppen oder mit einem Substituenten substituierte aromatische Gruppen und X Halogene darstellen) oder eine halogenierte Vinylverbindung oder ein Derivat davon der Formel

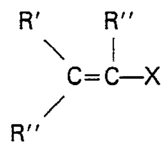

(worin R, R′ und R′′ die oben angegebenen Bedeutungen besitzen und X ein Halogen darstellt) verwendet wird und als halogensubstituierte aromatische Verbindung eine halogenierte Arylverbindung oder ein Derivat davon der Formel

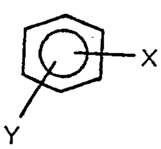

(worin X Halogene und Y ein Wasserstoffatom oder mindestens ein Substituent aus der Alkylgruppen oder Alkenylgruppen mit 5 oder weniger als 5 Kohlenstoffatomen, Cyanogruppen, Nitrogruppen, Estergruppen und Arylgruppen umfassenden Gruppe bedeuten) verwendet wird.

14. Verfahren nach einem der Ansprüche 12 oder 13, dadurch gekennzeichnet, daß als Katalysator des Palladium-Typs eine zweiwertige oder nullwertige Palladiumverbindung eingesetzt wird.

15. Verfahren nach einem der Ansprüche 11 bis 14, dadurch gekennzeichnet, daß $R_f$ eine fluorhaltige Alkylgruppe oder eine fluorhaltige Alkenylgruppe mit 10 oder weniger Kohlenstoffatomen bedeutet.

16. Verfahren nach Anspruch 11 oder 15, dadurch gekennzeichnet, daß R und R′ alkylgruppen oder Alkenylgruppen mit 15 oder weniger Kohlenstoffatomen bedeuten.

17. Verfahren nach einem der Ansprüche 11 bis 16, dadurch gekennzeichnet, daß ein fluorhaltiges, aliphatisches Iodid in Gegenwart eines aprotischen polaren Lösungsmittels mit einer Carbonylverbindung oder einer halogensubstituierten, aliphatischen oder aromatischen Verbindung umgesetzt wird.

18. Verfahren nach einem der Ansprüche 11 bis 17, dadurch gekennzeichnet, daß die Reaktion bei Umgebungstemperatur und Atmosphärendruck durchgeführt wird.

19. Verfahren nach einem der Ansprüche 11 bis 18, dadurch gekennzeichnet, daß das fluorhaltige, aliphatische Iodid der Formel

$$R_fI$$

(worin $R_f$ eine fluorhaltige, aliphatische Gruppe darstellt) mit Zinkpulver und/oder halogeniertem Zinnpulver, welches durch die Formel $SnX_2$ (worin X halogene darstellt) wiedergegeben werden kann, unter Erhitzen umgesetzt wird, um in dieser Weise $R_fZnI$ und/oder $R_fSnX_2I$ zu erzeugen, die dann für die Reaktion verwendet werden.

20. Verfahren nach Anspruch 19, dadurchgekennzeichnet, daß das Zinkpulver in einer Menge von 1 bis 3 Mol pro Mol $R_fI$ eingesetzt wird.

21. Verfahren nach Anspruch 1, welches ein Verfahren 1) gemäß dem ein halogeniertes Keton der Formel

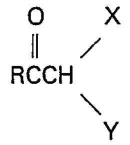

(worin R eine aliphatische Kohlenwasserstoffoxygruppe, eine aliphatische Kohlenwasserstoffgruppe oder eine fluoraliphatische Kohlenwasserstoffgruppe darstellt, die jene Gruppen umfaßt, die Ringe zwischen Kohlenstoffatomen aufweisen, an die X gebunden ist, X ein Halogen und Y Wasserstoffatome oder Fluoratome darstellen) mit einem Metall (M), wie Zink oder Magnesium, umgesetzt wird unter Bildung eines metallhalogenierten Ketons der Formel

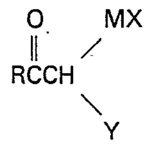

(worin R, X und Y die oben angegebenen Bedeutungen besitzen und M für Zink oder Magnesium steht); und ein Verfahren

2) gemäß dem das in dieser Weise gebildete metallhalogenierte Keton mit einem Fluorester der Formel

$$R_fCOOR'$$

(worin $R_f$ eine fluoraliphatische Kohlenwasserstoffgruppe darstellt, die auch solche mit Etherbindungen in ihrer Kette umfaßt; und R' eine aliphatische Kohlenwasserstoffgruppe oder eine aromatische Kohlenwasserstoffgruppe bedeutet) unter der Einwirkung von Ultraschallwellen umsetzt unter Bildung eines fluorhaltigen Diketons der Formel

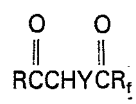

(worin R, Y und $R_f$ die oben angegebenen Bedeutungen besitzen).

22. Verfahren nach Anspruch 21, dadurch gekennzeichnet, daß ein halogeniertes Keton unter der Einwirkung von Ultraschallwellen in Gegenwart von Zink oder Magnesium mit einem Fluoralkylester umgesetzt wird.

23. Verfahren nach Anspruch 21, dadurch gekennzeichnet, daß ein halogeniertes Keton unter der Einwirkung von Ultraschallwellen mit Zink oder Magnesium umgesetzt wird unter Bildung eines metallhalogenierten Ketons, welches metall-halogenierte Keton unter der Einwirkung von Ultraschallwellen mit dem Fluoralkylester umgesetzt wird.

24. Verfahren nach Anspruch 21, dadurch gekennzeichnet, daß das metallhalogenierte Keton durch Umsetzen des halogenierten Ketons mit Zink oder Magnesium durch Erhitzen gebildet und dieses metallhalogenierte Keton unter der Einwirkung von Ultraschallwellen mit dem Fluoralkylester umgesetzt wird.

25. Verfahren nach einem der Ansprüche 21 bis 24, dadurch gekennzeichnet, daß die Reaktion in mindestens einem aprotischen polaren Lösungsmittel ausgewählt aus der Tetrahydrofuran, Dimethylformamid, Acetonitril, Dimethylsulfoxid, Dimethylacetamid, N-Methylpyrrolidon und Hexamethylphosphoramid umfassenden Gruppe, durchgeführt wird.

26. Verfahren nach einem der Ansprüche 21 bis 25, dadurch gekennzeichnet, daß die Anzahl der Kohlenstoffatome der Gruppe R auf 10 oder weniger begrenzt ist.

27. Verfahren nach Anspruch 26, dadurch gekennzeichnet, daß als halogeniertes Keton d-3-Halogen-Campher verwendet wird.

28. Verfahren nach einem der Ansprüche 21 bis 27, dadurch gekennzeichnet, daß X für Bromatome oder Iodatome steht.

29. Verfahren nach einem der Ansprüche 21 bis 28, dadurch gekennzeichnet, daß $R_f$ eine Fluoralkylgruppe oder -alkenylgruppe oder eine mit anderen Halogenen substituierte Fluoralkylgruppe oder -alkenylgruppe, die jeweils 15 oder weniger Kohlenstoffatome aufweisen, darstellt.

30. Verfahren nach einem der Ansprüche 21 bis 28, dadurch gekennzeichnet, daß $R_f$ eine Fluoralkylethergruppe der Formel

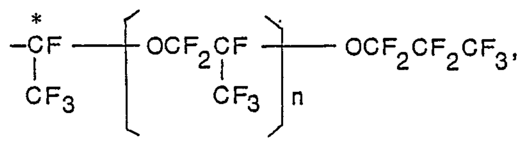

(worin n = 0 bis 3 und $\overset{*}{C}$ ein asymmetrisches Kohlenstoffatom bedeuten) darstellt.

31. Verfahren nach einem der Ansprüche 21 bis 30, dadurch gekennzeichnet, daß R' eine Alkylgruppe oder Alkenylgruppe mit 10 oder weniger Kohlenstoffatomen bedeutet.

**Revendications**

1. Un procédé pour la production de composés organiques contenant du fluor caractérisé en ce que l'on fait réagir des iodures aliphatiques contenant du fluor comprenant au moins un choix dans le groupe de $R_fI$, $R_fZnI$ et $R_fSnX_2I$ (où $R_f$ indique un groupe aliphatique contenant du fluor; et X indique un halogène) ou comprenant $R_fCOOR'$ (où $R_f$ indique un groupe aliphatique contenant du fluor; et R' indique un groupe hydrocarbure aliphatique ou un groupe hydrocarbure aromatique) avec des composés organiques sous l'action d'ondes ultrasonores, pour introduire le groupe $R_f$ ou le groupe $R_fCO$ dans lesdits composés organiques.

2. Un procédé selon la revendication 1 dans lequel on utilise comme dit composé organique un composé carbonylé qui est représenté par la formule: RCOR' (où R et R' représentent des atomes ou des groupes semblables ou différents qui sont choisis dans les groupes constitués par les atomes d'hydrogène, les groupes aliphatiques et les groupes aromatiques et lorsque tous deux sont le même groupe aliphatique, ils sont capables de former conjointement des cycles) et on le fait réagir avec ledit iodure aliphatique contenant du fluor en présence de poudre de zinc et/ou de poudre d'étain halogéné représenté par $SnX_2$ (où X représente des halogènes).

3. Un procédé selon la revendication 1 dans lequel ledit iodure aliphatique contenant du fluor est mis à réagir avec un composé insaturé halogéno-substitué ayant des liaisons multiples entre les atomes de carbone, ou un composé aromatique halogéno-substitué sous l'action d'ondes ultrasonores en présence de poudre de zinc et d'un catalyseur de type palladium pour introduire ledit $R_f$ dans ladite liaison multiple ou ledit noyau benzène et obtenir ainsi un composé aliphatique saturé ou un composé aromatique dont ledit halogène est éliminé.

4. Un procédé selon la revendication 3 dans lequel on utilise comme composé insaturé aliphatique halogéno-substitué un composé allylique halogéné, ou son dérivé, représenté par la formule:

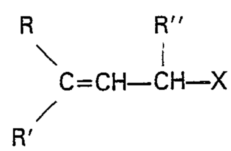

(où R, R' et R'' représentent un atome d'hydrogène, un groupe alcoyle ou un groupe alcényle, ayant un nombre d'atomes de carbone de 10 ou inférieur à 10, un groupe aromatique ou un groupe aromatique substitué par un substituant et X représente un halogène), ou un composé vinylique halogéné, ou son dérivé, représenté par la formule:

1

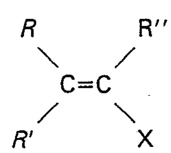

(où R, R' et R'' sont les mêmes que ceux décrits ci-dessus et X represente un halogène); et on utilise comme composé aromatique halogéno-substitué un composé arylique halogéné, ou son dérivé, représenté par la formule:

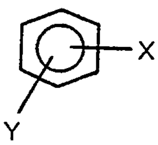

(où X représente des halogènes; et Y représente au moins un type d'un atome d'hydrogène ou de substituants qui sont choisis dans les groupes constitués par un groupe alcoyle ou un groupe alcényle ayant un nombre d'atomes de carbone de 5 ou inférieur à 5, ou un groupe cyano, un groupe nitro, un groupe ester et un groupe aryle).

5. Un procédé selon la revendication 3 ou la revendication 4 dans lequel un composé de palladium divalent ou zérovalent est utilisé comme catalyseur de type palladium.

6. Un procédé selon l'une quelconque des revendications 1 à 5, dans lequel $R_f$ est un groupe alcoyle ou un groupe alcényle contenant du fluor ayant un nombre d'atomes de carbone de 10 ou moins.

7. Un procédé selon la revendication 2 ou la revendication 6 dans lequel R et R' sont un groupe alcoyle ou un groupe alcényle ayant un nombre d'atomes de carbone de 15 ou moins.

8. Un procédé selon l'une quelconque de la revendication 1 à la revendication 7 dans lequel on utilise de la poudre de zinc et/ou de la poudre d'étain halogéné dans une gamme de 1 à 3 fois $R_fI$, en nombre de moles.

9. Un procédé selon l'une quelconque de la revendication 1 à la revendication 8, dans lequel on fait réagir en utilisant un solvant polaire aprotique un iodure aliphatique contenant du fluor et un composé carbonylique ou un composé aliphatique ou aromatique halogéno-substitué.

10. Un procédé selon l'une quelconque de la revendication 1 à la revendication 9, dans lequel on effectue la réaction à la température ambiante et à la pression atmosphérique.

11. Un procédé selon la revendication 1 dans lequel on fait réagir un iodure aliphatique contenant du fluor qui est représenté par la formule:

$$R_fZnI \text{ ou } R_fSnX_2I$$

(où $R_f$ représente des groupes aliphatiques contenant du fluor; et X représente des halogènes) avec un composé carbonylique qui est représenté par la formule:

$$RCOR'$$

(où R et R' représentent des atomes ou des groupes semblables ou différents qui sont choisis dans les groupes comprenant les atomes d'hydrogène, les groupes aliphatiques ou les groupes aromatiques et où lorsqu'ils sont tous deux des groupes aliphatiques, ils sont capables de former conjointement des cycles).

12. Un procédé selon la revendication 11 dans lequel ledit iodure de zinc aliphatique contenant du fluor est mis à réagir avec un composé insaturé aliphatique halogéno-substitué ayant des liaisons multiples entre des atomes de carbone ou un composé aromatique halogéno-substitué sous l'effet d'ondes ultrasonores et en présence d'un catalyseur de type palladium pour qu'un composé insaturé aliphatique ou un composé aromatique soit introduit avec ledit $R_f$ dans lesdites liaisons multiples ou ledit noyau benzène avec élimination dudit halogène.

13. Un procédé selon la revendication 12 dans lequel on utilise comme composé insaturé aliphatique halogéno-substitué un composé allylique halogéné, ou son dérivé, représenté par la formule:

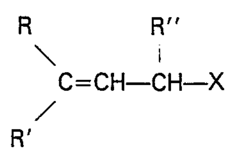

(où R, R' et R'' représentent un atome d'hydrogène, un groupe alcoyle ou un groupe alcényle, ayant un nombre d'atomes de carbone de 10 ou inférieur à 10, ou un groupe aromatique ou un groupe aromatique qui est substitué par un substituant; et X représente des halogènes), ou un composé vinylique halogéné, ou son dérivé, qui est représenté par la formule:

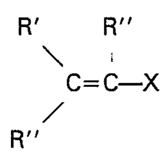

(où R, R' et R'' sont les mêmes que ceux précédemment décrits; et X représente des halogènes) et on utilise comme composé aromatique halogéno-substitué un composé arylique halogéné, ou son dérivé, qui est représenté par la formule:

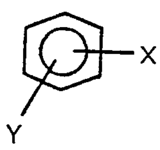

(ou X représente des halogènes; et Y représente un atome d'hydrogène ou au moins un substituant choisi dans les groupes comprenant un groupe alcoyle ou un groupe alcényle, ayant un nombre d'atomes de carbone de 5 ou inférieur à 5, un groupe cyano, un groupe nitro, un groupe ester et un groupe aryle.

14. Un procédé selon l'une de la revendication 12 ou de la revendication 13, dans lequel comme catalyseur de type palladium, on utilise un composé du palladium divalent ou zérovalent.

15. Un procédé selon l'une quelconque de la revendication 11 à la revendication 14, dans lequel $R_f$ représente un groupe alcoyle contenant du fluor ou un groupe alcényle contenant du fluor ayant un nombre d'atomes de carbone de 10 ou moins.

16. Un procédé selon la revendication 11 ou la revendication 15, dans lequel R et R' sont un groupe alcoyle ou un groupe alcényle ayant un nombre d'atomes de carbone de 15 ou moins.

17. Un procédé selon l'une quelconque des revendications 11 à 16, dans lequel on fait réagir un iodure aliphatique contenant du fluor avec un composé carbonylique ou un composé aliphatique ou aromatique halogéno-substitué avec emploi d'un solvant polaire aprotique.

18. Un procédé selon l'une quelconque de la revendication 11 à la revendication 17 dans lequel on effectue la réaction à la température ambiante et à la pression atmosphérique.

19. Un procédé selon l'une quelconque des revendications 11 à 18 dans lequel on fait réagir un iodure aliphatique contenant du fluor, qui est représenté par la formule:

$$R_fI$$

(où $R_f$ représente un groupe aliphatique contenant du fluor) avec de la poudre de zinc et/ou de la poudre d'étain halogéné qui est représenté par $SnX_2$ (où X représente des halogènes) à chaud, pour produire $R_fZnI$ et/ou $R_fSnX_2I$, le ou les produits étant ensuite utilisé(s) pour la réaction.

20. Un procédé selon la revendication 19 dans lequel on utilise la poudre de zinc dans la gamme de 1 à 3 fois $R_fI$, en nombre de moles.

21. Un procédé selon la revendication 1 ayant un procédé 1) dans lequel on fait réagir une cétone halogénée qui est représentée par la formule:

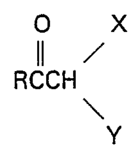

(où R représente un groupe hydrocarbure aliphatique-oxy, un groupe hydrocarbure aliphatique ou un groupe fluorohydrocarbure aliphatique et comprend ceux qui forment des cycles entre les atomes de carbone unis à X; X représente un halogène; et Y représente des atomes d'hydrogène ou des atomes de fluor) avec un métal (M) constitué du zinc ou du magnésium, pour former une cétone halogénée métallique qui est représentée par la formule:

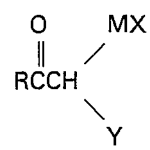

(dans laquelle R, X et Y sont comme ceux décrits précédemment et M représente la zinc ou le magnésium); et un procéde 2) dans lequel la cétone halogénée métallique ainsi formée est mise à réagir avec un fluoroester qui est représenté par la formule:

$R_fCOOR'$

(dans laquelle $R_f$ représente un groupe hydrocarbure fluoroaliphatique et comprend ceux ayant des liaisons éthers dans leurs chaînes; R' représente un groupe hydrocarbure aliphatique ou un groupe hydrocarbure aromatique) sous l'effet d'ondes ultrasonores pour former une dicétone contenant du fluor qui est représentée par la formule:

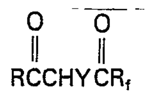

(où R, Y et $R_f$ sont comme ceux décrits précédemment).

22. Un procédé selon la revendication 21 dans lequel on fait réagir une cétone halogénée avec un ester fluoroalcoylique sous l'effet d'ondes ultrasonores en présence de zinc ou de magnésium.

23. Un procédé selon la revendication 21 dans lequel on fait réagir une cétone halogénée avec du zinc ou du magnésium sous l'effet d'ondes ultrasonores pour produire une cétone halogénée métallique et de plus on fait réagir cette cétone halogénée métallique avec un ester fluoroalcoylique sous l'effet d'ondes ultrasonores.

24. Un procédé selon la revendication 21 dans lequel on forme une cétone halogénée métallique par mise en réaction à chaud une cétone halogénée et de zinc ou de magnésium et de plus on soumet cette cétone halogénée métallique à une réaction avec un ester fluoro-alcoylique sous l'effet d'ondes ultrasonores.

25. Un procédé selon l'une quelconque de la revendication 21 à la revendication 24 dans lequel on fait s'effectuer la réaction dans au moins un solvant polaire aprotique choisi dans un groupe comprenant le tétrahydrofuranne, le diméthylformamide, l'acétonitrile, le diméthyl-sulfoxyde, le diméthylacétamide, la N-méthylpyrrolidone et l'hexaméthylphosphoramide.

26. Un procédé selon l'une quelconque de la revendication 21 à la revendication 25 dans lequel le nombre des atomes de carbone de R est limité à 10 ou moins.

27. Un procédé selon la revendication 26 dans lequel on utilise comme cétone halogénée un d-3-halogénocamphre.

28. Un procédé selon l'une quelconque de la revendication 21 à la revendication 27 dans lequel X est un atome de brome ou un atome d'iode.

29. Un procédé selon l'une quelconque de la revendication 21 à la revendication 28 dans lequel $R_f$ est un groupe fluoroalcoyle ou un groupe alcényle, ou un groupe fluoro-alcoyle ou un groupe alcényle qui sont substitués et dans lesquels d'autres halogènes sont introduits, et ayant tous un nombre d'atomes de carbone de 15 ou moins.

30. Un procédé selon l'une quelconque de la revendication 21 à la revendication 28 dans lequel $R_f$ est un groupe fluoro-alcoyléther qui est représenté par la formule:

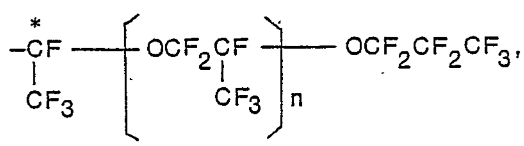

(où n = 0~3; et $\overset{*}{C}$ représente un carbone asymétrique).

31. Un procédé selon l'une quelconque de la revendication 21 à la revendication 30 dans lequel R' est un groupe alcoyle ou un groupe alcényle ayant un nombre d'atomes de carbone de 10 ou moins.